# EUROPÄISCHE PATENTSCHRIFT

(11) **EP 1 176 144 B1**
(45) Veröffentlichungstag und Bekanntmachung des Hinweises auf die Patenterteilung: **09.07.2003**
(21) Anmeldenummer: 01117433.1
(22) Anmeldetag: 19.07.2001
(51) Int. Cl.: C07D 403/14, C07D 403/06, A61K 31/496, A61P 1/00, A61P 25/00

(54) **Neue N-Triazolylmethyl-Piperazinderivate als Neurokininrezeptor-Antagonisten**
N-triazolylmethyl-piperazine derivatives as neurokinine receptor-antagonists
Dérivés de N-triazolylméthyl-piperazine, en tant qu' antagonistes récepteurs de la neuroquinine

(30) Priorität: 28.07.2000 DE 10036818
(43) Veröffentlichungstag der Anmeldung: 30.01.2002
(73) Patentinhaber: Solvay Pharmaceuticals GmbH, 30173 Hannover (DE)
(72) Erfinder: Jasserand, Daniel, 30625 Hannover (DE); Schön, Uwe, 31303 Burgdorf (DE); Sann, Holger, 30629 Hannover (DE); Brückner, Reinhard, 30559 Hannover (DE); Eeckhout, Christian, 29690 Lindwedel (DE)
(74) Vertreter: Bauriegel, Lutz

(56) Entgegenhaltungen:
- EP-A- 0 655 442
- EP-A- 0 899 270
- WO-A-97/22597
- WO-A-98/57954

## Beschreibung

Die vorliegende Erfindung betrifft neue, Neurokininrezeptor-antagonistisch wirksame 2-Indolylmethyl-Piperazinderivate, welche an einem Stickstoff des Piperazin-Grundgerüstes durch einen Triazolylmethylrest substituiert sind. Weiterhin betrifft die Erfindung diese neuen N-Triazolylmethyl-Piperazinderivate enthaltende Arzneimittel. Ferner betrifft die Erfindung ein Verfahren zur Herstellung der neuen Piperazinderivate sowie Zwischenprodukte dieses Verfahrens.

Aus der WO 98/57954 sind bereits zu den Verbindungen der vorliegenden Erfindung strukturell ähnliche Verbindungen bekannt, welchen allgemeine, auf Tachykinin, Neurokinin A oder auch Neurokinin B antagonistisch wirkende Eigenschaften zugeschrieben werden und welche in der Lage sind, das zentrale Nervensystem (= ZNS) zu beeinflussen.

In der EP 0 899 270 A1 werden 2-Indolylmethyl-Piperazinderivate mit einem zu den Verbindungen der vorliegenden Erfindung unterschiedlichen Substitutionsmuster beschrieben, welche Neurokininrezeptor-antagonistische Eigenschaften aufweisen.

Ferner sind aus der EP 0 655 442 A1 weitere 2-Indolylmethyl-Piperazinderivate mit einem zu den Verbindungen der vorliegenden Erfindung unterschiedlichen Substitutionsmuster bekannt, denen allgemein auf Tachykinin, Neurokinin A oder auch Neurokinin B antagonistisch wirkende Eigenschaften zugeschrieben werden und welche für geeignet angesehen werden, das ZNS zu beeinflussen.

Der vorliegenden Erfindung lag die Aufgabe zu Grunde, neue Wirkstoffe mit Neurokinin-(= NK)rezeptor-antagonistischen Eigenschaften und einem verbesserten Wirkungsprofil bereitzustellen, welche insbesondere zur Behandlung von peripheren Störungen wie funktionellen und entzündlichen Störungen des gastrointestinalen Traktes geeignet sind.

Es wurde nun überraschend gefunden, daß sich eine Gruppe von neuen N-Triazolylmethyl-Piperazinderivaten durch spezifische NK-1-Rezeptor-antagonistische Eigenschaften auszeichnet und ihre Wirkung vorzugsweise im peripheren Bereich entfaltet. Demgemäß erscheint die erfindungsgemäße Gruppe von Verbindungen besonders geeignet zur Behandlung von durch NK-1 induzierten peripheren Störungen, insbesondere zur Behandlung von funktionellen und entzündlichen Störungen des gastrointestinalen Traktes.

Gegenstand der Erfindung sind daher neue Verbindungen der allgemeinen Formel I, 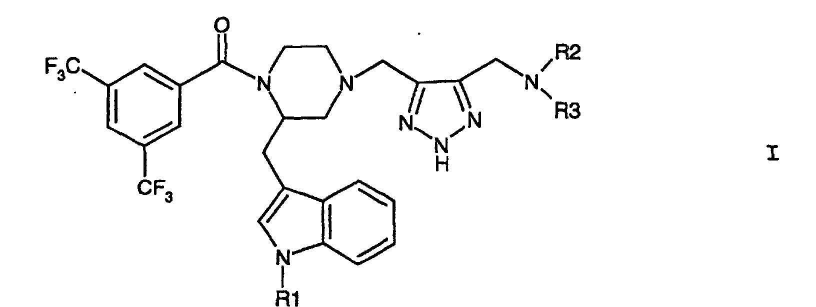 worin
- R¹: Wasserstoff oder Niederalkyl bedeutet,
- R²: Niederalkyl, Diniederalkylaminoniederalkyl, Niederalkoxycarbonylniederalkyl, gegebenenfalls ein- oder zweifach durch Niederalkyl substituiertes Cyclo(hetero)alkyl mit 5 - 6 Ringatomen, welches gegebenenfalls 1 - 2 Doppelbindungen enthält, gegebenenfalls im (Hetero)phenylring ein- oder zweifach durch Halogen, Niederalkyl und/oder Niederalkoxy substituiertes (Hetero)phenylniederalkyl, dessen Niederalkylkette gegebenenfalls ein- oder zweifach durch Niederalkyl oder durch Spiro-C₄-C₅-alkylen substituiert ist, oder im Phenylring gegebenenfalls ein- oder zweifach durch Halogen, Niederalkyl und/oder Niederalkoxy substituiertes Phenylniederalkoxy bedeutet und
- R³: Niederalkyl, Niederalkoxycarbonylniederalkyl oder gegebenenfalls ein- oder zweifach durch Niederalkyl substituiertes Cyclo(hetero)alkyl mit 5 - 6 Ringatomen bedeutet, oder
- R² und R³: gemeinsam mit dem Stickstoff, an welchen sie gebunden sind, eine cyclische Gruppe der Formel a bilden, 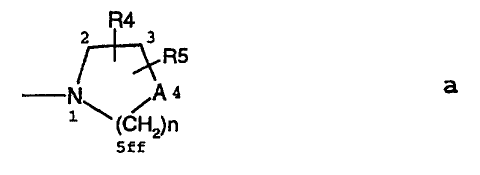 worin
A Stickstoff, Sauerstoff, Methylen oder Methyliden, dessen Doppelbindung gemeinsam mit dem benachbarten Kohlenstoff in 3-Position der Gruppe a gebildet wird, bedeutet,
n eine ganze Zahl von 1 bis 3 bedeutet,
R⁴ Wasserstoff, Niederalkyl, Niederalkoxyniederalkyl, Niederalkoxycarbonyl, Niederalkoxycarbonylniederalkyl, Diniederalkylaminoniederalkyl, gegebenenfalls ein- oder zweifach durch Halogen, Niederalkyl und/oder Niederalkoxy substituiertes (Hetero)phenyl, gegebenenfalls im (Hetero)phenylring ein - oder zweifach durch Halogen, Niederalkyl und/oder Niederalkoxy substituiertes (Hetero)phenylniederalkyl, dessen Niederalkylkette gegebenenfalls einoder zweifach durch Niederalkyl substituiert ist, Cyclo(hetero)alkyl mit 5 - 6 Ringatomen oder Cyclo(hetero)alkylniederalkyl, dessen Cyclo(hetero)alkylgruppe 5 - 6 Ringatome aufweist, bedeutet und
R⁵ Wasserstoff, Niederalkyl oder Niederalkoxyniederalkyl bedeutet, oder
R⁴ und R⁵ gemeinsam an ein Kohlenstoff der Gruppe a gebundenes Spiroethylendioxy, an zwei benachbarte Atome der Gruppe a gebundenes C₃-C₄-Alkylen oder über zwei benachbarte Kohlenstoffe der Gruppe a anelliertes Phenyl bedeuten, oder
- R² und R³: gemeinsam mit dem Stickstoff, an welchen sie gebunden sind, einen Pyrrolidinring bilden, welcher zweifach durch jeweils über zwei benachbarte Kohlenstoffatome gebundenes C₄-Alkylen substituiert ist,
sowie physiologisch verträgliche Säureadditionssalze von Verbindungen der Formel I. Weiterhin sind Gegenstand der Erfindung die Verbindungen der Formel I enthaltende Arzneimittel. Ferner sind Gegenstand der Erfindung ein Verfahren zur Herstellung der Verbindungen der Formel I sowie Zwischenprodukte dieses Verfahrens.

Sofern in den Verbindungen der Formel I die Substituenten niedere Alkyl- oder Alkoxygruppen bedeuten oder enthalten, können diese geradkettig oder verzweigt sein und 1 bis 4 Kohlenstoffatome enthalten. Sofern die Substituenten Halogen enthalten, kommen insbesondere Fluor, Chlor oder Brom, vorzugsweise Fluor oder Chlor in Frage.

Sofern die Substituenten der Verbindungen der Formel I Cyclo(hetero)alkyl bedeuten oder enthalten, kann dieses für reine Carbocyclen stehen, oder kann auch Carbocyclen darstellen, worin jeweils 1 bis 3 Ringkohlenstoffatome durch Stickstoff, Sauerstoff und/oder Schwefel ersetzt sind. Stickstoff und Sauerstoff sind als Heteroatome bevorzugt. Sofern die Substituenten der Verbindungen der Formel I (Hetero)phenyl bedeuten oder enthalten, kann dieses für Phenyl stehen, oder kann auch Phenyl, worin jeweils 1 bis 3 Ringkohlenstoffatome durch Stickstoff ersetzt sind, darstellen. Sofern die Substituenten der Verbindungen der Formel I Heterophenyl bedeuten oder enthalten, steht dieses für Phenyl, worin jeweils 1 bis 3 Ringkohlenstoffatome durch Stickstoff ersetzt sind.

R¹ steht vorzugsweise für Wasserstoff. Sofern R¹ für Niederalkyl steht, ist Methyl bevorzugt.

R² steht vorzugsweise für Niederalkyl, insbesondere Methyl, Ethyl, Isopropyl oder tert.-Butyl; für Diniederalkylaminoniederalkyl, insbesondere Dimethylaminoethyl oder Dimethylamino-n-propyl; für Niederalkoxycarbonylniederalkyl, insbesondere Ethoxycarbonylmethyl; für gegebenenfalls einfach durch Niederalkyl, insbesondere Methyl, substituiertes Cyclo(hetero)alkyl mit 5 bis 6 Ringatomen, insbesondere für gegebenenfalls substituiertes Cyclopentyl, Cyclohexyl oder Piperidinyl; für gegebenenfalls im Heterophenylring ein- oder zweifach durch Niederalkyl, insbesondere Methyl, oder durch Niederalkoxy, insbesondere Methoxy, substituiertes Heterophenylniederalkyl, insbesondere für gegebenenfalls substituiertes Pyridyl; oder für im Phenylring ein- oder zweifach durch Niederalkyl, insbesondere Methyl, oder durch Niederalkoxy, insbesondere Methoxy, substituiertes Phenyl-C₂-C₄-alkyl.

R³ steht vorzugsweise für Niederalkyl, insbesondere Methyl, Ethyl oder Isopropyl; oder für Niederalkoxycarbonylniederalkyl, insbesondere Ethoxycarbonylmethyl.

Bevorzugt sind solche Verbindungen der Formel I, worin R² und R³ nicht gleichzeitig Isobutyl bedeuten.

Sofern R² und R³ gemeinsam mit dem Stickstoff, an welchen sie gebunden sind, eine Gruppe der Formel a bilden, steht R⁴ vorzugsweise für Wasserstoff; für Niederalkyl, insbesondere Methyl oder Isopropyl; für Niederalkoxyniederalkyl, insbesondere Methoxymethyl; für Niederalkoxycarbonylniederalkyl, insbesondere Ethoxycarbonylmethyl; für Diniederalkylaminoniederalkyl, insbesondere Dimethylaminoethyl; für gegebenenfalls einfach durch Niederalkyl, insbesondere Methyl, oder durch Niederalkoxy, insbesondere Methoxy, substituiertes (Hetero)phenyl, insbesondere für gegebenenfalls substituiertes Phenyl, Pyridyl, Pyrimidyl oder Pyrazolyl; für gegebenenfalls im (Hetero)phenylring einfach durch Halogen, Niederalkyl, insbesondere Methyl, oder durch Niederalkoxy, insbesondere Methoxy, substituiertes (Hetero)phenylniederalkyl, insbesondere für gegebenenfalls substituiertes Benzyl oder Pyridylniederalkyl; für Cyclo(hetero)alkyl mit 5 bis 6 Ringatomen, insbesondere für Cyclohexyl, Pyrrolidinyl oder Piperidinyl; oder für Cyclo(hetero)alkylniederalkyl, dessen Cyclo(hetero)alkylring 5 bis 6 Ringatome aufweist, insbesondere für Pyrrolidinyl-C₁-C₂-alkyl, Morpholinoethyl oder Cyclohexylmethyl.

In der allenfalls vorhandenen Gruppe der Formel a steht R⁵ vorzugsweise für Wasserstoff; für Niederalkyl, insbesondere Methyl; oder für Niederalkoxyniederalkyl, insbesondere Methoxymethyl.

Bevorzugt sind solche Verbindungen der Formel I, worin in der allenfalls vorhandenen Gruppe der Formel a R⁴ und R⁵ nicht an dasselbe Atom der Gruppe a gebunden sind, mit Ausnahme der bevorzugten Verbindungen der Formel I, worin R⁴ und R⁵ gemeinsam an einen Kohlenstoff der Gruppe a gebundenes Spiroethylendioxy bedeuten. Ebenfalls sind Verbindungen der Formel I bevorzugt, worin R⁴ und R⁵ an zwei benachbarte Ringatome der Gruppe a gebundenes C₃-C₄-Alkylen bedeuten.

In der allenfalls vorhandenen Gruppe der Formel a steht n für eine ganze Zahl von 1 bis 3. Sofern R⁴ und R⁵ beide Wasserstoff bedeuten und A gleichzeitig für Methylen steht, steht n vorzugsweise für 2 oder 3.

Allgemein können die Substituenten R⁴ und R⁵ der Gruppe a an jedes Ringatom der Gruppe gebunden sein, einschließlich der durch A gebildeten Ringatome, welche nicht für Sauerstoff stehen. Sofern ein Ringatom der Gruppe a durch R⁴ oder R⁵ substituiert ist, tritt R⁴ oder R⁵ an die Stelle eines andernfalls an derselben Stelle vorhandenen Wasserstoffatoms, so daß die üblichen Bindungswertigkeiten der Ringatome der Gruppe a erhalten bleiben. Sofern A für Methyliden steht, wird dessen Doppelbindung vorzugsweise mit dem benachbarten Kohlenstoff in 3-Position der Gruppe a gebildet, welcher in diesem Fall ebenfalls eine Methylidengruppe bildet.

Bevorzugt sind Verbindungen der Formel I, worin eine gegebenenfalls vorhandene Gruppe a für durch R⁴ und R⁵ substituiertes Pyrrolidin steht, wobei R⁴ und R⁵ nicht beide gleichzeitig Wasserstoff bedeuten, oder worin eine gegebenenfalls vorhandene Gruppe a für jeweils durch R⁴ und R⁵ substituiertes 2,5-Dihydropyrrol, Piperidin, Piperazin, Morpholin oder Diazepan steht.

Besonders bevorzugt ist die Verbindung (2R)-1-[3,5-Bis(trifluormethyl)benzoyl]-2-(lH-indol-3-ylmethyl)-4-{[5-(morpholinomethyl)-2H-1,2,3-triazol-4-yl]methyl}piperazin der Formel I.

Die Verbindungen der Formel I sowie deren Säureadditionssalze können hergestellt werden, indem man
a) eine Verbindung der allgemeinen Formel II, 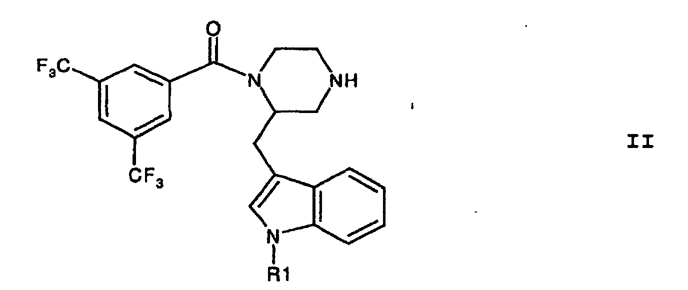 worin R¹ obige Bedeutung besitzt, mit einer Verbindung der allgemeinen Formel III, 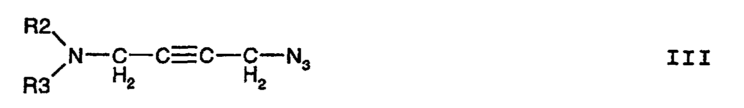 worin R² und R³ obige Bedeutungen besitzen, und worin allenfalls vorhandene reaktive Gruppen durch geeignete Schutzgruppen blockiert sind, umsetzt, oder
b) eine Verbindung der allgemeinen Formel IV 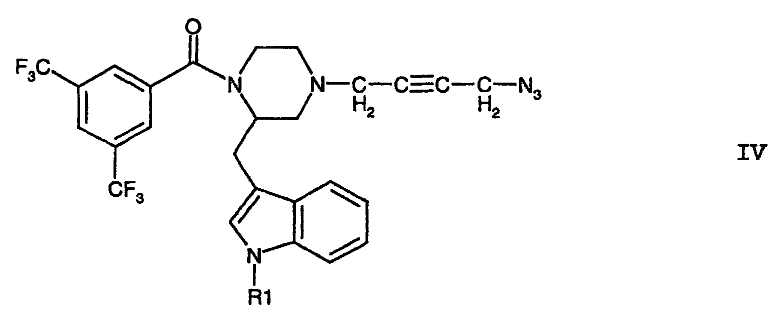 worin R¹ obige Bedeutung besitzt, mit einer Verbindung der allgemeinen Formel V, 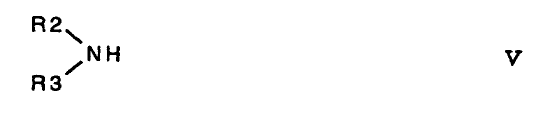 worin R² und R³ obige Bedeutungen besitzen, und worin allenfalls vorhandene reaktive Gruppen durch geeignete Schutzgruppen blockiert sind, umsetzt,
und allenfalls vorhandene Schutzgruppen nachfolgend wieder abspaltet und eine erhaltene Verbindung der Formel I gewünschtenfalls in ihr Säureadditionssalz überführt oder ein Säureadditionssalz in eine freie Verbindung der Formel I überführt.

Gemäß Verfahrensvariante a) kann eine sekundäre Aminofunktion eines substituierten Piperazinderivates der Formel II mit einem N,N-disubstituierten Azidobutinamin der Formel III umgesetzt werden, um eine Verbindung der Formel I zu erhalten. Die Reaktion kann in einem unter den Reaktionsbedingungen inerten Lösungsmittel wie einem dipolar-aprotischen Lösungsmittel, beispielsweise Ethylacetat oder Dimethylformamid (= DMF), oder vorzugsweise in einem Gemisch solcher Lösungsmittel durchgeführt werden. Besonders bevorzugt ist ein Gemisch aus Ethylacetat und DMF. Geeignete Reaktionstemperaturen liegen zwischen Raumtemperatur und der Siedetemperatur des Lösungsmittels bzw. des Lösungsmittelgemisches. Sofern Verbindungen der Formel III eingesetzt werden, welche zusätzliche, unter den Reaktionsbedingungen reaktive funktionelle Gruppen aufweisen, werden diese zusätzlichen funktionellen Gruppen zweckmäßigerweise durch an sich bekannte Schutzgruppen blockiert. Geeignete Schutzgruppen, welche nach an sich bekannten Methoden eingeführt und später nach an sich bekannten Methoden wieder abgespalten werden können, sind beispielsweise bekannt aus J. A. W. McOmie, "Protective Groups in Organic Chemistry", Plenum Press, 1973, oder aus T. W. Green und P. G. M. Wuts, "Protective Groups in Organic Synthesis", Wiley and Sons, 1991. Der Fachmann kann geeignete Schutzgruppen jeweils durch Routinemethoden auswählen.

Die Verbindungen der Formel II und deren stereoisomere Formen sind aus der EP 0 655 442 A1 bekannt und können nach den in dieser Patentanmeldung beschriebenen Verfahren oder analog zu diesen Verfahren hergestellt werden.

Verbindungen der Formel III können hergestellt werden, indem man eine Verbindung der allgemeinen Formel VI, 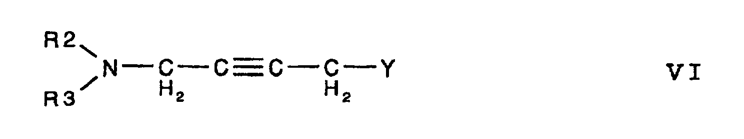 worin R² und R³ obige Bedeutungen besitzen und worin Y für eine abspaltbare Fluchtgruppe steht, mit einem Alkalimetallazid, vorzugsweise Natriumazid, auf an sich zur Azidbildung bekannte Weise umsetzt. Als Fluchtgruppe Y kommt insbesondere Halogen, vorzugsweise Chlor, oder es kommen auch Sulfonyloxygruppen, welche gute Fluchtgruppen bilden, beispielsweise Niederalkansulfonyloxy wie Methansulfonyloxy, oder gegebenenfalls im Benzolring durch Halogen oder niederes Alkyl substituiertes Benzolsulfonyloxy wie p-Toluolsulfonyloxy, in Frage.

Verbindungen der Formel VI können hergestellt werden, indem man eine Verbindung der Formel V mit einer Verbindung der allgemeinen Formel VII, 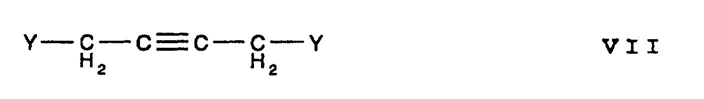 worin Y obige Bedeutung besitzt, auf an sich bekannte Weise umsetzt. Erhaltene Verbindungen der Formel VI können gewünschtenfalls noch gereinigt werden. Zur Reinigung können Verbindungen der Formel VI beispielsweise in geeignete Salze wie Oxalate überführt und nach an sich bekannten Kristallisationsverfahren gereinigt werden. Die vorgenannten Salze der Verbindungen der Formel VI können auch direkt für weitere Umsetzungen eingesetzt werden.

Sekundäre Amine der Formel V sind an sich bekannt. Die Verbindungen der Formel VII sind ebenfalls an sich bekannt. Vorzugsweise kann als Verbindung der Formel VII das 1,4-Dichlor-2-butin verwendet werden.

Gemäß Verfahrensvariante b) kann ein Piperazin-N-butinazid der Formel IV mit einem sekundären Amin der Formel V umgesetzt werden, um eine Verbindung der Formel I zu erhalten. Die Reaktion kann auf an sich bekannte Weise, beispielsweise nach einem aus K. Banert, Chemische Berichte 122 (1989) 1963-1967 bekannten oder dazu analogen Verfahren, in einem unter den Reaktionsbedingungen inerten Lösungsmittel wie Ethylacetat oder einem Ether, beispielsweise Tetrahydrofuran (= THF) oder Dioxan, durchgeführt werden. Sofern anwendbar, kann in einer bevorzugten Verfahrensvariante das sekundäre Amin der Formel V selbst, beispielsweise Morpholin, als Lösungsmittel verwendet werden. Eine geeignete Reaktionstemperatur muß in Abhängigkeit vom verwendeten sekundären Amin der Formel V gewählt werden. Sofern das Amin der Formel V bei Raumtemperatur flüssig oder fest ist, kann üblicherweise bei Temperaturen zwischen Raumtemperatur und dem Siedepunkt üblicher Lösungsmittel, beispielsweise bis etwa 100 °C, gearbeitet werden. Sofern leichtflüchtige Amine der Formel V eingesetzt werden, beispielsweise Dimethylamin oder Diethylamin, wird zweckmäßigerweise bei tiefen Temperaturen gearbeitet, beispielsweise zwischen -78 °C und -10 °C und vorzugsweise bei erhöhtem Druck, beispielsweise bei 1,5 bis 3 bar. Reaktionen unter erhöhtem Druck können in an sich bekannten Autoklaven durchgeführt werden. Sofern Amine der Formel V eingesetzt werden, welche zusätzliche, unter den Reaktionsbedingungen reaktive funktionelle Gruppen aufweisen - beispielsweise eine zweite sekundäre Aminofunktion, sofern Piperazin verwendet wird - werden diese zusätzlichen funktionellen Gruppen zweckmäßigerweise durch an sich bekannte Schutzgruppen blockiert. Geeignete Schutzgruppen, welche nach an sich bekannten Methoden eingeführt und später nach an sich bekannten Methoden wieder abgespalten werden können, sind beispielsweise bekannt aus den in Verfahrensvariante a) vorgenannten Publikationen. Sofern Piperazin als Amin der Formel V verwendet wird, ist die tert.-Butoxycarbonylgruppe als Schutzgruppe bevorzugt. Der Fachmann kann geeignete Schutzgruppen jeweils durch Routinemethoden auswählen.

Verbindungen der Formel IV sind neue Verbindungen, welche sich als Zwischenprodukte zur Herstellung neuer Wirkstoffe, beispielsweise zur Herstellung der Verbindungen der Formel I, eignen. Die Verbindungen der Formel IV können hergestellt werden, indem man Verbindungen der allgemeinen Formel VIII, 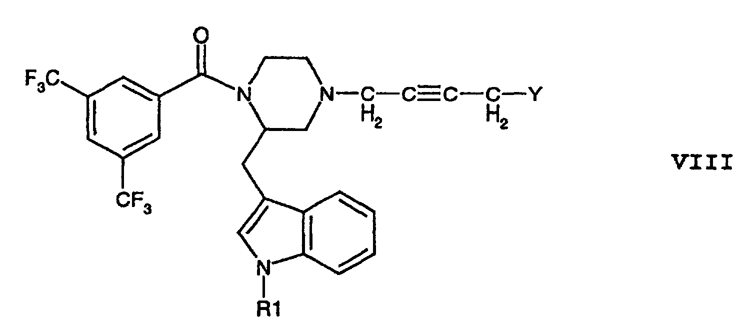 worin R¹ und Y obige Bedeutungen besitzen, mit einem Alkalimetallazid, vorzugsweise Natriumazid, auf an sich zur Azidbildung bekannte Weise umsetzt.

Verbindungen der Formel VIII können hergestellt werden, indem man Verbindungen der Formel II mit Verbindungen der Formel VII auf an sich bekannte Weise umsetzt.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in physiologisch verträgliche Säureadditionssalze überführt werden.

Als physiologisch verträgliche Salze von Verbindungen der Formel I kommen deren Salze mit anorganischen Säuren, beispielsweise Schwefelsäure, Phosphorsäuren oder Halogenwasserstoffsäuren, vorzugsweise Chlorwasserstoffsäure, oder mit organischen Säuren, beispielsweise niederen aliphatischen Mono-, Di- oder Tricarbonsäuren wie Maleinsäure, Fumarsäure, Milchsäure, Weinsäure, Zitronensäure, oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, in Frage. Ein bevorzugtes Salz einer Verbindung der Formel I ist das (2R)-1-[3,5-Bis(trifluormethyl)benzoyl]-2-(1H-indol-3-ylmethyl)-4-{[5-(morpholinomethyl)-2H-1,2,3-triazol-4-yl]methyl}piperazin-dihydrochlorid, da es vergleichsweise gut wasserlöslich ist.

Die Verbindungen der Formel I enthalten jedenfalls ein chirales Kohlenstoffatom, nämlich das den 1H-Indol-3-ylmethylrest tragende Kohlenstoffatom in 2-Stellung des Piperazin-Grundgerüstes. Die Verbindungen der Formel I können somit in mehreren stereoisomeren Formen vorliegen. Die vorliegende Erfindung umfaßt sowohl die Gemische optischer Isomere als auch die isomerenreinen Verbindungen der Formel I. Bevorzugt sind Verbindungen der Formel I, worin der Indolylmethylrest in 2R-Stellung des Piperazinringes angeordnet ist. Falls bei der Synthese der Verbindungen der Formel I Gemische optischer Isomere der Ausgangsverbindung, beispielsweise der Verbindungen der Formel II oder der Verbindungen der Formel IV eingesetzt werden, werden auch die Verbindungen der Formel I in Form von Gemischen optischer Isomere erhalten. Ausgehend von stereochemisch einheitlichen Formen der Ausgangsverbindung können auch stereochemisch einheitliche Verbindungen der Formel I erhalten werden. Die stereochemisch einheitlichen Verbindungen der Formel I können aus den Gemischen optischer Isomere in an sich bekannter Weise erhalten werden, z. B. durch chromatographische Trennung an chiralen Trennmaterialien oder durch Umsetzung mit geeigneten optisch aktiven Säuren, beispielsweise Weinsäure oder Campher-10-sulfonsäure, und anschließender Auftrennung in die optisch aktiven Antipoden durch fraktionierte Kristallisation der gewonnenen diastereomeren Salze.

In den Verbindungen der Formel I kann der 1,2,3-Triazolring in mehreren tautomeren Formen vorliegen, so daß das Wasserstoffatom an verschiedene Atome des 1,2,3-Triazolringes gebunden sein kann. Im Rahmen der vorliegenden Erfindung sollen die Verbindungen der Formel I alle möglichen Tautomere des Triazolringes mitumfassen.

Die Verbindungen der Formel I und ihre Säureadditionssalze besitzen Neurokinin(= NK)-Rezeptor-antagonistische Eigenschaften und sind zur Behandlung von Krankheitszuständen bei größeren Säugetieren, insbesondere Menschen, geeignet, bei denen Neurokinine als Überträgerstoffe beteiligt sind. Dabei zeichnet sich die erfindungsgemäße Gruppe von Verbindungen durch ein besonders günstiges Wirkprofil aus, welches gekennzeichnet ist durch eine hohe selektive Affinität zu NK-1-Rezeptoren. Weiterhin zeichnet sich die erfindungsgemäße Gruppe von Verbindungen durch eine gute Verträglichkeit auch über längere Applikationszeiträume und durch eine vergleichsweise gute orale Bioverfügbarkeit aus.

Auf Grund ihres Wirkprofils und ihres selektiven und reversiblen Bindungsvermögens an NK-1-Rezeptoren eignet sich die erfindungsgemäße Gruppe von Verbindungen insbesondere zur Hemmung von Vorgängen, an denen an NK-1-Rezeptoren bindende Neurokinine wie Substanz P beteiligt sind. Somit eignen sich die Verbindungen selektiv zur Behandlung von Krankheitszuständen, bei welchen Substanz P beteiligt ist. Substanz P spielt beispielsweise eine Rolle bei der Schmerzübertragung, Emese, neurogenen Entzündungen, Harnblasenentzündungen, entzündlichen Gelenkserkrankungen und asthmatischen Beschwerden. Wegen der in vorteilhafter Weise auf den peripheren Bereich gerichteten Wirkung eignet sich das Wirkprofil der Verbindungen für die Behandlung von peripheren pathologischen Störungen, insbesondere zur Behandlung von funktionellen und entzündlichen Störungen im gastrointestinalen Trakt. Zu den durch die erfindungsgemäßen Verbindungen behandelbaren funktionellen Störungen zählen insbesondere die als sogenanntes "irritable bowel syndrome" (= IBS) oder Reizdarmsyndrom bekannten Störungen der unteren Darmwege. Wesentliche Symptome von IBS sind Unterbauchschmerzen, die auf einer Übersensibilität des visceralen afferenten Nervensystems zu beruhen scheinen, und Anomalien des Stuhlganges, insbesondere anomal beschleunigte Passage des Stuhls im Colon. Die erhöhte viscerale Schmerzempfindlichkeit gegenüber mechanischen oder chemischen Reizen im intestinalen Trakt führt dazu, daß IBS-Patienten schon bei physiologischen verdauungsbedingten geringen Dehnungen des Colons, z. B. bereits bei geringer Gasbildung und leichten Blähungen, die von Gesunden kaum wahrgenommen werden, starke viscerale Schmerzen erleiden. Zu durch die erfindungsgemäßen Verbindungen günstig beeinflußbaren inflammatorisch bedingten Störungen im gastrointestinalen Trakt gehören die im allgemeinen unter dem Begriff IBD (= inflammatory bowel disease) zusammengefaßten entzündlichen Störungen im Dünndarm- und Dickdarmbereich, u. a. Colitis ulcerosa und Morbus Crohn. Das Wirkprofil der Verbindungen zeichnet sich durch vergleichsweise gute orale Bioverfügbarkeit mit einer günstigen Selektivität der Neurokinin-Rezeptor-antagonistischen Wirkungen gegenüber bei insbesondere auf den peripheren Bereich abzielenden Therapieansätzen unerwünschten Begleitwirkungen aus. So wurde in Dosisbereichen, die den NK-1-Rezeptor blockieren, in pharmakologischen Testversuchen keine cardiovasculäre calciumantagonistische Wirkung festgestellt. Weiterhin kann von den erfindungsgemäßen Verbindungen angenommen werden, daß sie keine nennenswerten zentralnervösen Begleitwirkungen entfalten.

### Beschreibung der pharmakologischen Testmethoden

Die angegebenen Beispielsnummern der in den pharmakologischen Tests als Testsubstanzen eingesetzten Verbindungen beziehen sich auf die nachstehend beschriebenen Herstellungsbeispiele.

### 1. Bestimmung des Bindungsvermögens der Testsubstanzen an NK-1-Rezeptoren in vitro.

Die Affinität der Testsubstanzen zu humanen NK-1-Rezeptoren wird in vitro gemessen. Bestimmt wird die Hemmung der Bindung des physiologischen Neurokinins (= Substanz P) an Neurokinin-1-Rezeptoren.

Die Rezeptor-Bindungsstudien werden mit [³H]-Substanz P als Ligand durchgeführt. Für den Bindungsversuch werden verschiedene Proben einer Membranpräparation von CHO-Zellen (= Eizellen des chinesischen Hamsters, chinese hamster oocytes), die den humanen NK-1-Rezeptor ("Accession Number" der zugehörigen Nukleinsäuresequenz = M74290; "Accession Number" der zugehörigen Proteinsequenz = P25103; vgl. Takeda, Y.; Chou, K. B., Takeda, J.; Sachais, B.S. und Krause, J. E; Biochemical and Biophysical Research Communications, 179(3) (1991) 1232 - 1240) exprimieren, mit einer Lösung des markierten Liganden inkubiert, wobei die Inkubationsansätze keine Testsubstanz oder Zusätze unterschiedlicher Konzentrationen an Testsubstanz enthalten. Anschließend werden in den Proben gebundene und freie Liganden jeweils durch Glasfiber-Filtration voneinander getrennt. Die im Filter verbleibende Fraktion wird mehrmals mit Pufferlösung gewaschen und anschließend wird die Radioaktivität der im Filter verbliebenen Fraktion mit einem Beta-Scintillationszähler gemessen.

Für die Verbindungen der Beispiele 1 sowie 8 bis 65 wurde die Affinität zu humanen NK-1-Rezeptoren jeweils durch einmalige Messung der Testsubstanzen in einer Konzentration von 10⁻⁷ mol/l bestimmt. Alle vorgenannten Testsubstanzen zeigten in diesem Testmodell eine Verdrängung des physiologischen NK-Rezeptor-Liganden Substanz P von ≥75 %. Die Verbindungen der Beispiele 1, 8-15, 17-29, 34-47, 49-55, 57, 59-60 und 62-65 zeigten eine Verdrängung von jeweils ≥90 %.

Für die Verbindungen der Beispiele 2 und 4 - 6 wird als IC₅₀ der jeweiligen Testsubstanz diejenige Konzentration bestimmt, welche eine halbmaximale Verdrängung des gebundenen Liganden bewirkt. Aus dieser wird die entsprechende Inhibitionskonstante (Kᵢ-Wert) der Testsubstanz berechnet und als negativer dekadischer Logarithmus des Kᵢ-Wertes (= pKᵢ-Wert) angegeben. Der pKᵢ-Wert ist ein Maß für die Affinität der Testsubstanzen zu humanen NK-1-Rezeptoren. In diesem Testmodell zeigten die in der folgenden Tabelle 1 aufgeführten Testsubstanzen die angegebenen pKᵢ-Werte:

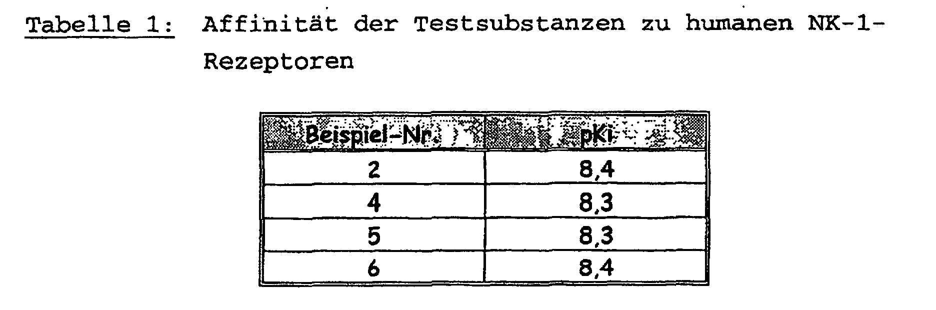

### 2. Bestimmung des funktionellen NK-1-Antagonismus der Testsubstanzen an isoliertem Gewebe vom Meerschweinchen in vitro

Die NK-1-Rezeptor-antagonistische Wirkung der Testsubstanzen wurde an isolierten, in einer oxygenierten Nährlösung gehaltenen Ringpräparaten der Aorta von Pirbright-White Meerschweinchen in vitro gemessen. Bestimmt wird die Hemmung der nach Stimulierung mit dem NK-1-Agonisten Substanz P hervorgerufenen Tonusrelaxation der Aortapräparate durch die Testsubstanzen.

Zur Messung der Kontraktion der Gefäßmuskulatur werden die Präparate an einem Haken fixiert, durch einen Faden mit einem Kraftmessungsgerät verbunden und die Kontraktionen werden jeweils an einem Schreiber registriert. Die Aortapräparate werden mit Phenylephrin tonisiert. Anschließend werden die NK-1-Rezeptoren der Präparate vor und nach der Gabe der Testsubstanz mit 0,01 µmol Substanz P stimuliert, wodurch eine Relaxation des Tonus herbeigeführt wird. Die Relaxationen vor und nach Gabe der Testsubstanz werden in Prozent quantifiziert. Es wird die effektive Konzentration der halbmaximalen Hemmung der Tonusrelaxation (= EC₅₀) errechnet. Als Kenngröße wird der negative dekadische Logarithmus des EC₅₀-Wertes (= pEC₅₀) angegeben. Der pEC₅₀-Wert ist ein Maß für die funktionelle Wirksamkeit der Testsubstanzen an NK-1-Rezeptoren. In diesem Testmodell zeigten die in der folgenden Tabelle 2 aufgeführten Testsubstanzen die angegebenen pEC₅₀-Werte:

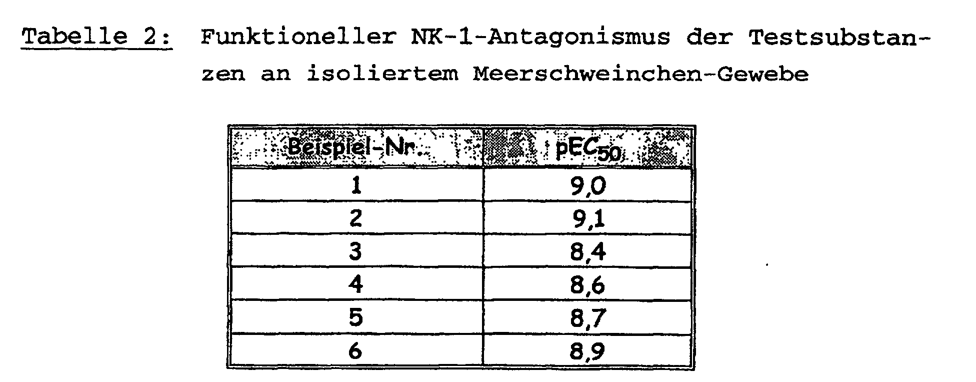

### 3. Bestimmung der Substanz-P-antagonistischen Wirkung der Testsubstanzen in vivo.

Zum Nachweis der Substanz-P-antagonistischen Wirkung der Testsubstanzen wurde als Standardtestmodell für Substanz-Pinduzierte pharmakologische Effekte die durch intravenöse (= i.v.) Substanz-P-Applikation hervorgerufene transiente Hypotension in Meerschweinchen verwendet. Es wurde die Hemmwirkung der Testsubstanzen gegenüber durch Substanz P induzierter Blutdrucksenkung einerseits nach i.v. und andererseits nach oraler (= p. o.) Applikation der Testsubstanzen bestimmt.

Männlichen Meerschweinchen werden unter Narkose (Ketamin 67 mg/ kg, Xylazine 13 mg/kg) jeweils ein Katheter in eine Arteria Carotis Communis und in eine Vena Jugularis implantiert. Der arterielle Katheter dient zur Blutdruckmessung. Über den venösen Zugang erfolgt die Gabe von Substanz P bzw. bei i.v.-Gabe auch die Applikation der Testsubstanz. Nach einer Äquilibrierungsphase von 20 Minuten werden 50 pmol/Tier Substanz P (Bolus, i. v.) als Teststimulus appliziert. Eine Minute nach Applikation des Teststimulus wird jeweils die hierdurch induzierte maximale Blutdrucksenkung als Kontrolle für die spätere Stimulation der NK-1-Rezeptoren durch die Testsubstanz ermittelt. Danach wird die Testsubstanz gegeben. Bei der i. v. Untersuchung wird die Testsubstanz in Dosierungen von 0.01 bis 0.1 µmol/kg verabreicht. Bei der p. o.-Untersuchung wird die Testsubstanz in Dosierungen von 0,1 bis 3,2 µmol/kg verabreicht. Als Vehikel dient bei den p. o.-Untersuchungen Tylose bzw. Tylose/Ethanol. Anschließend wird jeweils das Ausmaß der durch die Testsubstanz inhibierten Blutdrucksenkung, beginnend mit 1 Minute nach Testsubstanzgabe, bis zu einer Dauer von 90 Minuten nach Testsubstanzgabe, in Abständen von jeweils 15 Minuten gemessen. Aus diesen Meßwerten werden als ED₅₀-Werte diejenigen Dosen bestimmt, bei welchen in Abhängigkeit von der Zeit gerade eine 50%ige Hemmung der Substanz-P-induzierten Blutdrucksenkung durch die Testsubstanz eintritt. Als Kenngröße wird der negative dekadische Logarithmus der ED₅₀ (= pED₅₀) angegeben.

In diesem Testmodell zeigte die Testsubstanz des Beispiels 1 eine Stunde nach i. v.-Applikation einen pED₅₀-Wert von 7,6. Dieselbe Testsubstanz des Beispieles 1 zeigte eine Stunde nach p. o.-Applikation einen pED₅₀-Wert von 6,2. Die Verbindung des Beispiels 6 zeigte eine Stunde nach i. v.-Applikation einen pED₅₀-Wert von 7,0. Diese Werte belegen die hohe pharmakologische Potenz der Verbindungen der Formel I, insbesondere auch bei oraler Applikation.

In dem gleichen Testmodell werden die Testsubstanzen auch auf auf calciumantagonistischen Eigenschaften beruhende blutdrucksenkende Wirkungen untersucht. Hierzu wird die Wirkung der Testsubstanzen auf den basalen Blutdruck untersucht. Die Substanz des Beispiels 1 zeigte in dem untersuchten Dosisbereich (i. v.-Dosen bis zu 0,1 µmol/kg und p. o.-Dosen bis zu 3,2 µmol/kg) keine signifikante Blutdrucksenkung. Dies ist ein Indiz dafür daß in diesem Dosisbereich keine caiciumantagonistischen Nebenwirkungen auftraten. Die geringen calciumantagonistischen Begleitwirkungen der erfindungsgemäßen Verbindungen lassen sich auch durch in vitro-Standardtestmodelle, beispielsweise an isoliertem Ileum-Gewebe von Meerschweinchen, nachweisen.

In einem Standardtest zur Ermittlung ZNS-permeabler Verbindungen mit NK-1-antagonistischer Wirkung ("gerbil foot tapping test", vgl. N. M. Rupniak, A. R. Williams, European Journal of Pharmacology 265 (1994) 179-183), zeigte die Verbindung des Beispiels 1 selbst in hohen Dosierungen von bis zu 30 mg/kg p. o. keine für ZNS-permeable NK-1-Antagonisten typischen Effekte.

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 80 mg, insbesondere 1 bis 10 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nachfolgend angeführten Beispiele sollen die Erfindung näher erläutern, ohne sie in ihrem Umfang zu beschränken.

### Beispiel 1

### (2R)-1-[3,5-Bis(trifluormethyl)benzoyl]-2-(1H-indol-3-ylmethyl)-4-{[5-(morpholinomethyl)-2H-1,2,3-triazol-4-yl]-methyl}piperazin

### Syntheseweg 1

A) Zu einer Suspension von 43 g K₂CO₃ in 100 ml DMF wurden bei 20 °C unter Schutzgasatmosphäre 22 ml 1,4-Dichlor-2-butin zugegeben. Man erhitzte das Gemisch auf 50 °C und tropfte anschließend eine Lösung von 50 g (2R)-1-[3,5-Bis(trifluormethyl)benzoyl]-2-(1H-indol-3-ylmethyl)piperazin in 200 ml DMF zu dieser Vorlage zu. Das erhaltene Gemisch wurde 5 Stunden lang bei 50 °C gerührt. Man ließ über Nacht bei Raumtemperatur weiter rühren, filtrierte vom ungelösten Niederschlag ab und wusch den Niederschlag zweimal mit je 200 ml Ethylacetat nach. Die vereinigten Filtrate wurden im Vakuum zur Trockene eingedampft und das erhaltene Öl wurde durch Säulenchromatographie (Kieselgel; Laufmittel: n-Hexan/Ethylacetat 60/40 bis 30/70) gereinigt. Die vereinigten Produktfraktionen wurden erneut zur Trockene eingedampft. Man erhielt 40,4 g (2R)-1-[3,5-Bis (trifluormethyl)benzoyl]2-(1H-indol-3-ylmethyl]-4-(4-chlor-2-butin-1-yl)piperazin als amorphen gelblichen Feststoff, welcher ohne weitere Reinigung für die nächste Synthesestufe eingesetzt wurde.
B) Zu einer Lösung von 40,0 g des vorstehend unter 1A) erhaltenen Chlorbutinyl-Piperazinderivates in 200 ml Dimethylsulfoxid (= DMSO) wurden bei Raumtemperatur und unter Schutzgasatmosphäre 5,76 g NaN₃ zugegeben. Das entstandene Gemisch wurde für 24 Stunden unter Schutzgasatmosphäre bei Raumtemperatur weiter gerührt. Zu dieser Vorlage gab man eine Lösung von 50 g Ammoniumchlorid in 300 ml Wasser hinzu. Anschließend wurde die wäßrige Phase mit 500 ml Methyl-tert.-butylether (= MTBE) extrahiert. Man wusch die organische Phase nacheinander mit 200 ml gesättigter Kochsalzlösung und 200 ml Wasser, trocknete über Natriumsulfat und dampfte schließlich im Vakuum zur Trockene ein. Der zurückbleibende gelbliche Schaum wurde durch Säulenchromatographie (Kieselgel; Laufmittel: Ethylacetat/n-Hexan 65/35 bis 80/20) gereinigt. Die vereinigten Produktfraktionen wurden erneut zur Trockene eingedampft. Man erhielt 33,2 g (2R)-1-[3,5-Bis(trifluormethyl)benzoyl)-2-(1Hindol-3-ylmethyl)-4-[4-azido-2-butin-1-yl]piperazin als gelblichen Feststoff, welcher ohne weitere Reinigung für die nächste Synthesestufe eingesetzt wurde.
C) 25,5 g des vorstehend unter 1B) erhaltenen Azido-Derivates wurden in 10 ml Morpholin gelöst und 4 Stunden lang unter Schutzgasatmosphäre auf 80 °C erhitzt. Man ließ das Reaktionsgemisch bei Raumtemperatur über Nacht weiter rühren und dampfte anschließend im Vakuum zur Trockene ein. Der Rückstand wurde in 500 ml Ethylacetat aufgenommen und erneut im Vakuum zur Trockene eingedampft. Der erhaltene Schaum wurde durch Säulenchromatographie (Kieselgel; Laufmittel: Ethylacetat/Ethanol 100/0 bis 85/15) gereinigt. Die vereinigten Produktfraktionen wurden zur Trockene eingedampft. Man erhielt 22,0 g der Titelverbindung als gelblichen amorphen Feststoff, Fp.= 92° bis 98 °C (Glasübergangstemperatur), [α]_{D}²⁰ = 5° (c = 1,0 in Methanol).
D) Zu einer Lösung von 39,0 g der vorstehend unter 1C) erhaltenen Titelverbindung in 100 ml MTBE wurden unter Schutzgasatmosphäre 20 ml Ethanol abs. zugegeben. Die erhaltene Reaktionsmischung wurde auf 40° bis 50 °C erhitzt und man gab zu dieser Vorlage 81 ml einer 1.6 N HCl in Isopropanol dazu. Das Reaktionsgemisch wurde weitere 10 Minuten lang bei 50 °C gerührt. Anschließend gab man langsam 1.000 ml MTBE zu, woraufhin das Salz auszufallen begann. Man ließ zur Vervollständigung der Fällung noch 2 Stunden lang bei Raumtemperatur weiter rühren, filtrierte von der flüssigen Phase ab und wusch das Salz zweimal mit MTBE. Nach Trocknung im Vakuum erhielt man 39,5 g der festen Titelverbindung als weißes bis beiges Dihydrochlorid, Fp. = 213° bis 216 °C; [α]_{D}²⁰ = -3,6° (c = 1,0 in Methanol).

### Syntheseweg 2

A) 17,0 ml 1,4-Dichlor-2-butin wurden unter Schutzgasatmosphäre in 100 ml Toluol gelöst und die Lösung wurde mit 42,6 g K₂CO₃ versetzt. Nach Erwärmung der entstandenen Suspension auf 50 °C ließ man zu dieser Vorlage langsam eine Lösung von 10 ml Morpholin in 100 ml Toluol zutropfen. Das entstandene Reaktionsgemisch wurde noch 5 Stunden lang bei 50 °C und anschließend über Nacht bei Raumtemperatur gerührt. Es wurde vom K₂CO₃ abfiltriert, zweimal mit je 100 ml Toluol nachgewaschen und die vereinigten Filtrate wurden im Vakuum eingeengt. Der Rückstand wurde mit etwa 100 ml Toluol aufgenommen und die organische Phase wurde der Reihe nach mit gesättigten wäßrigen Lösungen von NaHCO₃ und NaCl gewaschen. Die organische Phase wurde im Vakuum zur Trockene eingedampft. Man erhielt 14,3 g rohes 1-(4-Chlor-2-butin-1-yl)morpholin als Öl, welches zur Reinigung in das Monooxalat überführt wurde:
   Zur Reinigung wurden 14,0 g der vorstehend erhaltene Rohverbindung in 80 ml MTBE aufgenommen. Man filtrierte vom ausgefallenen Niederschlag ab und wusch zweimal mit je 100 ml MTBE nach. Die vereinigten Filtrate wurden unter Schutzgasatmosphäre auf 50 °C erwärmt. Zu dieser Vorlage wurde eine 50 °C warme Lösung von 10,0 g Oxalsäure in 40 ml Ethanol zugegeben. Nach Abkühlung auf Raumtemperatur wurde das Reaktionsgemisch noch über Nacht gerührt. Man filtrierte das entstandene Oxalat ab und wusch den Feststoff noch dreimal mit je 20 ml MTBE nach. Es wurden 13,0 g [1-(4-Chlor-2-butin-1-yl)morpholin]-monooxalat erhalten, Fp. = 144 - 146 °C.
B) 510 mg des vorstehend unter Beispiel 1/Syntheseweg 2A) erhaltenen Oxalsäure-Salzes wurden bei Raumtemperatur unter Schutzgasatmosphäre in 10 ml DMF gelöst. Zu dieser Vorlage gab man 154 mg NaN₃ zu und ließ 10 Minuten lang rühren. Anschließend wurden 0,6 ml Triethylamin zugegeben. Die entstandene Suspension wurde noch 15 Stunden lang weiter gerührt und das so entstandene 1-(4-Azido-2-butin-1-yl)morpholin wurde direkt in der Suspension ohne weitere Aufarbeitung oder Charakterisierung für die nächste Synthesestufe eingesetzt.
C) Die vorstehend unter Beispiel 1/Syntheseweg 2B) erhaltene Suspension der Azido-Verbindung wurde mit 60 ml Ethylacetat verdünnt. Zu dieser Vorlage gab man 1,5 ml einer 55-Gew.-%igen Lösung von (2R)-1-[3,5-Bis(trifluormethyl)-benzoyl]-2-(1H-indol-3-ylmethyl)piperazin in THF zu, erhitzte das entstandene Gemisch 15 Stunden lang zum Sieden und ließ anschließend noch 3 Tage lang bei Raumtemperatur weiter rühren. Die resultierende Lösung wurde 3 mal mit je 50 ml Wasser gewaschen und die organische Phase wurde über Natriumsulfat getrocknet. Der nach Eindampfen und Trocknung im Hochvakuum verbliebene Rückstand wurde durch kombinierte Flüssigkeitschromatographie/Massenspektroskopie (= LC/MS) als die Titelverbindung identifiziert.

### Beispiel 2:

### (2R)-1-[3,5-Bis(trifluormethyl)benzoyl]-2-(1H-indol-3-ylmethyl)-4-{[5-(piperazinomethyl)-2H-1,2,3-triazol-4-yl)]-methyl}piperazin

A) 1,0 g (2R)-1-[3,5-Bis(trifluormethyl)benzoyl]-2-(lH-indol-3-ylmethyl)-4-[4-azido-2-butin-1-yl]piperazin (Herstellung siehe Beispiel 1/Syntheseweg 1B)) wurden unter Schutzgasatmosphäre in 50 ml Ethylacetat gelöst. Zu dieser Vorlage gab man 0,4 g tert.-Butoxycarbonylpiperazin und erhitzte das entstandene Reaktionsgemisch 8 Stunden lang unter Rückflußkühlung auf 80 °C. Man ließ über Nacht bei Raumtemperatur rühren und wusch die organische Phase anschlieβend dreimal mit je 10 ml Wasser. Die organische Phase wurde über Natriumsulfat getrocknet und im Vakuum zur Trockene eingedampft. Man erhielt 1,4 g (2R)-1-[3,5-Bis(trifluormethyl)benzoyl]-2-(1-methyl-indol-3-ylmethyl)-4-{[5-(4-tert.-butoxycarbonylpiperazin-1-yl)-2H-1,2,3-triazol-4-yl]methyl}piperazin als gelbbraunen Schaum, der ohne weitere Reinigung direkt für die nächste Synthesestufe eingesetzt wurde.
B) 330 mg der vorstehend unter 2A) erhaltenen Verbindung wurden in 10 ml Methanol gelöst. Zu dieser Vorlage wurden 10 ml eine 1,5 N HCl in Isopropanol zugegeben und das entstandene Reaktionsgemisch wurde über Nacht bei Raumtemperatur gerührt. Anschließend gab man eine Lösung von 0,4 g NaOH in 10 ml Wasser zu. Es wurde im Vakuum bis fast zur Trockene eingedampft und der Rückstand wurde mit 50 ml CH₂Cl₂ extrahiert. Die organische Phase wurde mit 100 ml Wasser gewaschen und im Vakuum zur Trockene eingedampft. Man erhielt 270 mg eines Salzsäuresalzes der Titelverbindung als Schaum, Fp. >200 °C.

### Beispiel 3:

### (2R)-1-[3,5-Bis(trifluormethyl)benzoyl]-2-(1-methyl-indol-3-methyl)-4-{[5-(morpholinomethyl)-2H-1,2,3-triazol-4-yl)]-methyl}piperazin

A) Zu einer Suspension von 7,4 g K₂CO₃ in 150 ml THF wurden zuerst 50 ml einer 55 Gew.-%igen Lösung von (2R)-1-[3,5-Bis(trifluormethyl)benzoyl]-2-(1H-indol-3-ylmethyl)-piperazin in THF und anschließend 25 ml Wasser zugegeben. Zu dieser Vorlage gab man eine Lösung von 12,2 g tert.-Butoxycarbonylanhydrid in 50 ml THF zu und ließ 12 Stunden lang bei Raumtemperatur rühren. Nach Einengen der Reaktionsmischung im Vakuum wurde der Rückstand in 300 ml MTBE aufgenommen und die organische Phase wurde der Reihe nach zweimal mit je 100 ml Wasser, einmal mit 50 ml einer 15 Gew.-%igen wäßrigen Weinsäurelösung und noch viermal mit je 100 ml Wasser gewaschen und anschließend über Natriumsulfat/ SiO₂ getrocknet. Nach Abfiltrieren vom Trockenmittel wurde das Filtrat im Vakuum eingeengt und der Rückstand wurde in 20 ml MTBE aufgenommen. Nach Erwärmung auf 60 °C wurden 120 ml Ligroin zugegeben und das Volumen wurde anschließend durch Vakuumdestillation um ca. 100 ml vermindert. Nach erneuter Zugabe von 100 ml Ligroin wurde zur Ausfällung 3 Tage lang stehen gelassen. Man filtrierte den ausgefallenen Feststoff ab, wusch noch dreimal mit je 30 ml Ligroin nach und trocknete bei 60 °C im Vakuum. Man erhielt 82,0 g (2R)-1-[3,5-Bis(trifluormethyl)benzoyl]-2-(1H-indol-3-ylmethyl)-4(tert.-butoxycarbonyl)piperazin als Feststoff, Fp.= 155 - 156 °C.
B) 5,0 g des vorstehend unter 3A) erhaltenen BOC-geschützten Piperazinderivates wurden bei Raumtemperatur unter Schutzgasatmosphäre in 100 ml trockenem DMF gelöst. Zu dieser Vorlage gab man 0,2 g NaH (60 %-ig in Mineralöl) zu und ließ 10 Minuten lang weiter rühren. Anschließend tropfte man eine Lösung von 1,9 ml CH₃I in 5 ml DMF zu und ließ nach vollendeter Zugabe 4 Stunden lang bei Raumtemperatur weiter rühren. Das Reaktionsgemisch wurde auf ein Gemisch aus 100 g Eis, 18 g Na₂S₂O₃ und 50 ml Wasser gegossen. Man extrahierte die wäßrige Phase mit einem Gemisch aus 200 ml Ethylacetat und 100 ml MTBE, wusch die organische Phase einmal mit Wasser und dampfte im Vakuum zur Trockene ein. Der ölige Rückstand wurde in 100 ml Methanol aufgenommen, mit 50 ml Diethylether gewaschen und erneut zur Trockene eingedampft. Man erhielt 5,5 g (2R)-1-[3,5-Bis(trifluormethyl)benzoyl]-2-(1-methyl-indol-3-ylmethyl)-4-(tert.butoxycarbonyl)piperazin als glasigen Feststoff, der ohne Reinigung oder Charakterisierung direkt für die nächste Synthesestufe eingesetzt wurde.
C) 5,9 g des vorstehend unter 3B) erhaltenen Indolyl-N-methylierten Piperazinderivates wurden in 60 ml Methanol gelöst. Zu dieser Vorlage gab man insgesamt 10 ml einer wäßrigen 1 N HCl langsam zu und ließ 48 Stunden lang bei Raumtemperatur rühren. Das Reaktionsgemisch wurde in 100 ml Methanol aufgenommen und zweimal mit je 20 ml n-Hexan gewaschen. Die Methanol-Phase wurde im Vakuum eingeengt und der Rückstand wurde in einem Gemisch aus 100 ml Wasser und 100 ml MTBE aufgenommen und über Nacht stehengelassen. Anschließend wurde die organische Phase als Überstand von der wäßrigen Phase abdekantiert und 3 mal mit je 30 ml 0,1 N wäßriger HCl extrahiert. Man neutralisierte die organische Phase mit einer gesättigten wäßrigen Lösung von K₂CO₃, woraufhin eine erste Fraktion (2R)-1-[3,5-Bis(trifluormethyl)benzoyl]-2-(1-methyl-indol-3-ylmethyl)piperazin als amorpher Feststoff ausfiel, welcher durch Überführung in das Oxalat und anschließende Neutralisation zurück zur Base gereinigt wurde. Durch Neutralisation der wäßrigen Phase wurde eine weitere Feststoff-Fraktion erhalten. Die vereinigten Feststoff-Fraktionen, welche durch Filtration aus der organischen und aus der wäßrigen Phase erhalten wurden, wurden im Hochvakuum getrocknet. Man erhielt insgesamt 4,6 g des vorgenannten entschützten Indolyl-N-methylierten Piperazinderivates.
D) 0,52 ml 1,4-Dichlor-2-butin wurden mit 1,2 g des vorstehend unter 3C) erhaltenen entschützten Indolyl-N-methylierten Piperazinderivates wie unter Beispiel 1/Syntheseweg 1A) beschrieben, umgesetzt. Nach Chromatographie (Kieselgel, Laufmittel: Ethylacetat/n-Hexan 65/35 bis 80/20) erhielt man 840 mg (2R)-1-[3,5-Bis(trifluormethyl)benzoyl]-2-(1-methyl-indol-3-ylmethyl]-4-(4-chlor-2-butin-1-yl) als Schaum, welcher ohne weitere Reinigung für die nächste Synthesestufe eingesetzt wurde.
E) 820 mg des vorstehend unter 3D) erhaltenen Chlorbutinyl-Piperazinderivates wurden mit 130 mg NaN₃ wie unter Beispiel 1/Syntheseweg 1B), beschrieben, umgesetzt. Man erhielt 760 mg (2R)-1-[3,5-Bis(trifluormethyl)benzoyl]-2-[1-methyl-indol-3-ylmethyl]-4-[4-azido-2-butin-1-yl]piperazin als Schaum, welcher ohne weitere Reinigung für die nächste Synthesestufe eingesetzt wurde.
F) 740 mg der vorstehend unter 3E) erhaltenen Azido-Verbindung wurden in 15 ml Morpholin gelöst. Das Reaktionsgemisch wurde wie unter Beispiel 1/Syntheseweg 1 C), beschrieben aufgearbeitet. Nach Chromatographie (Kieselgel, Laufmittel: CH₂Cl₂/Ethanol) erhielt man 470 mg der Titelverbindung als weißen Feststoff, welcher ohne weitere Reinigung für die nächste Synthesestufe eingesetzt wurde.
G) 440 mg der vorstehend unter 3F) erhaltenen Titelverbindung wurden mit 1 ml einer 1.6 N HCl in Isopropanol wie unter Beispiel 1/Syntheseweg 1D) beschrieben, in das HCl-Salz überführt. Man erhielt 425 mg des Dihydrochlorid-Monohydrates der Titelverbindung als Feststoff,
   Fp. = 192° - 200 °C.

### Beispiel 4:

### (2R)-1-[3,5-Bis(trifluormethyl)benzoyl]-2-(1H-indol-3-ylmethyl)-4-{[5-(dimethylaminomethyl)-2H-1,2,3-triazol-4-yl]-methyl}piperazin

660 mg (2R)-1-[3,5-Bis(trifluormethyl)benzoyl)-2-(1H-indol-3-ylmethyl)-4-[4-azido-2-butin-1-yl]piperazin (Herstellung siehe Beispiel 1/Syntheseweg 1B)) wurden in einem auf -20 °C vorgekühlten Autoklaven gegeben. Hierzu gab man eine auf -20 °C gekühlte Lösung von 2 Mol Dimethylamin in 30 ml THF zu. Nach Verschließen des Autoklaven wurde einen Tag lang bei 70 °C und bei einem Druck von 2,0 bis 2,2 bar gerührt. Anschließend ließ man unter ansonsten unveränderten Bedingungen noch über Nacht bei Raumtemperatur weiterrühren. Man dampfte im Vakuum zur Trockene ein und erhielt 700 mg der Titelverbindung als Schaum.

Zur Salzbildung wurden 680 mg der Titelverbindung in 10 ml Methanol gelöst. Zu dieser Vorlage wurden 1,5 ml einer 1.5 N HCl in Isopropanol zugegeben und es wurde anschließend im Hochvakuum zur Trockene eingedampft. Der Rückstand wurde zweimal mit je 20 ml Methanol aufgenommen und jeweils erneut zur Trockene eingedampft. Der zurückbleibende Feststoff wurde in 10 ml MTBE suspendiert und unter Rückflußkühlung 2 Stunden lang zum Sieden erhitzt. Nach Abkühlung auf Raumtemperatur und anschließender Filtration wurde der feste Niederschlag dreimal mit je 10 ml MTBE gewaschen und im Hochvakuum getrocknet. Man erhielt 670 mg eines Dihydrochlorid-dihydratsalzes der Titelverbindung als amorphen Feststoff, der u. a. durch Elementaranalyse charakterisiert wurde.

Nach den in vorstehenden Beispielen beschriebenen oder nach hierzu analogen Verfahren können auch die in der nachfolgenden Tabelle 3 angegebenen Verbindungen der Formel I hergestellt werden. Sofern in den nachfolgenden Tabellen 3 und 4 die Substituenten R² und R³ gemeinsam für "a" stehen, bilden R² und R³ gemeinsam mit dem Stickstoff, an welchen sie gebunden sind, eine vorstehend (s. Seite 3) beschriebene cyclische Gruppe der Formel a, worin R⁴, R⁵ A und n die jeweils in den Tabellen angegebenen Bedeutungen besitzen:

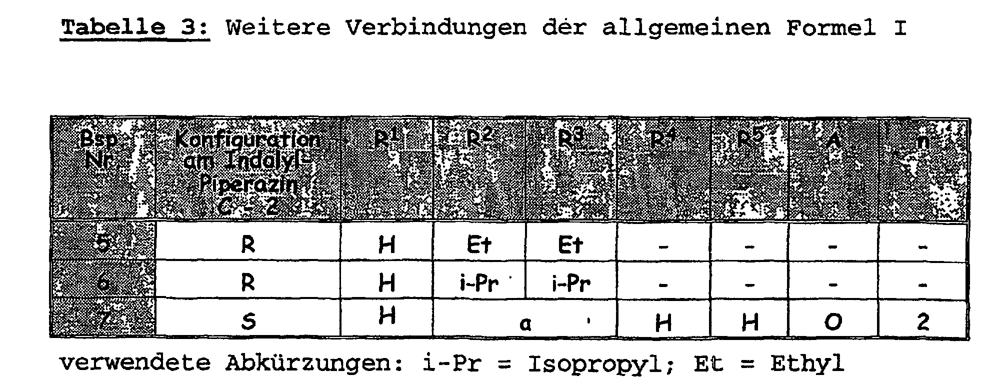

Die nachfolgenden Verbindungen der Beispiele 8 bis 65 wurden nach einem automatisierten Herstellungsverfahren hergestellt.

Hierzu wurde pro Ansatz eine Lösung von 0,03 mmol (2R)-1-[3,5- Bis(trifluormethyl)benzoyl]-2-(1H-indol-3-ylmethyl)-4-(4-azido-2-butin-1-yl)piperazin der Formel IV (Herstellung siehe Beispiel 1/Syntheseweg 1B)) in 1 ml Ethylacetat jeweils mit einer Lösung von 0,03 mmol des als Reaktionspartner vorgesehenen sekundären Amins der Formel V in 1 ml Ethylacetat umgesetzt und anschließend mit 3 ml Ethylacetat verdünnt. Auf das Reaktionsgemische wurde Stickstoffgas gegeben und es wurde jeweils 6 Stunden lang bei 70 °C gerührt. Der Reaktionsendpunkt wurde Dünnschicht-chromatographisch bestimmt. Nach vollendeter Umsetzung wurden die einzelnen Reaktionsgemische jeweils im Vakuum zur Trockene eingedampft. Aus dem Rückstand wurde ohne weitere Aufreinigung jeweils eine Probe für die Hochleistungs-Flüssigkeitschromatographie (= HPLC) und für die automatische Massenspektroskopie zur Bestimmung der Reinheit bzw. zur Strukturbestätigung entnommen.

Nach dem vorstehend angegebenen automatisierten Herstellungsverfahren können die in der nachfolgenden Tabelle 4 angegebenen Verbindungen der Formel I hergestellt werden. Alle in der Tabelle 4 angegebenen Verbindungen der Formel I weisen an dem den Indolylmethylrest tragenden Kohlenstoff C-2 des Piperazingerüstes die R-Konfiguration auf.

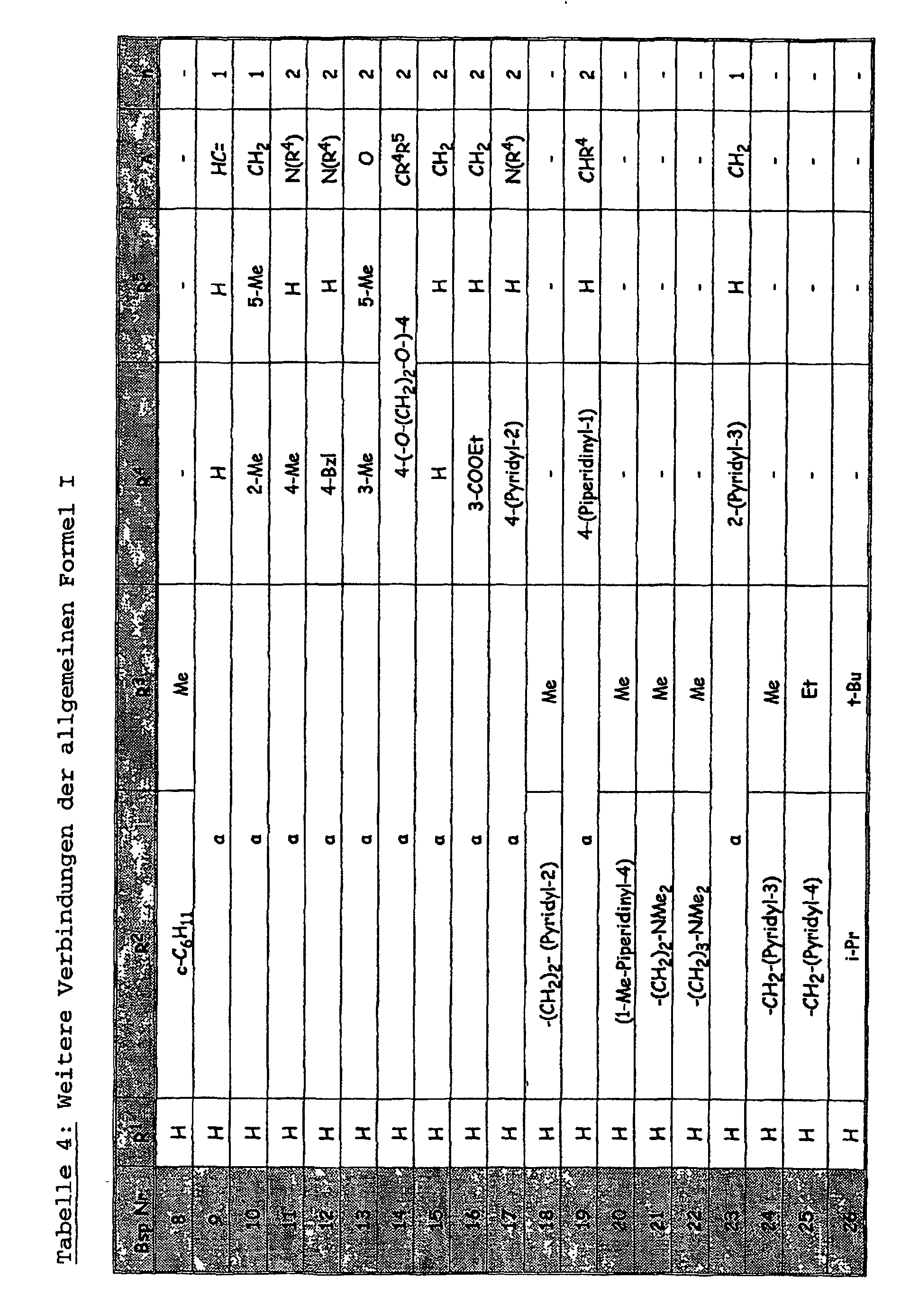

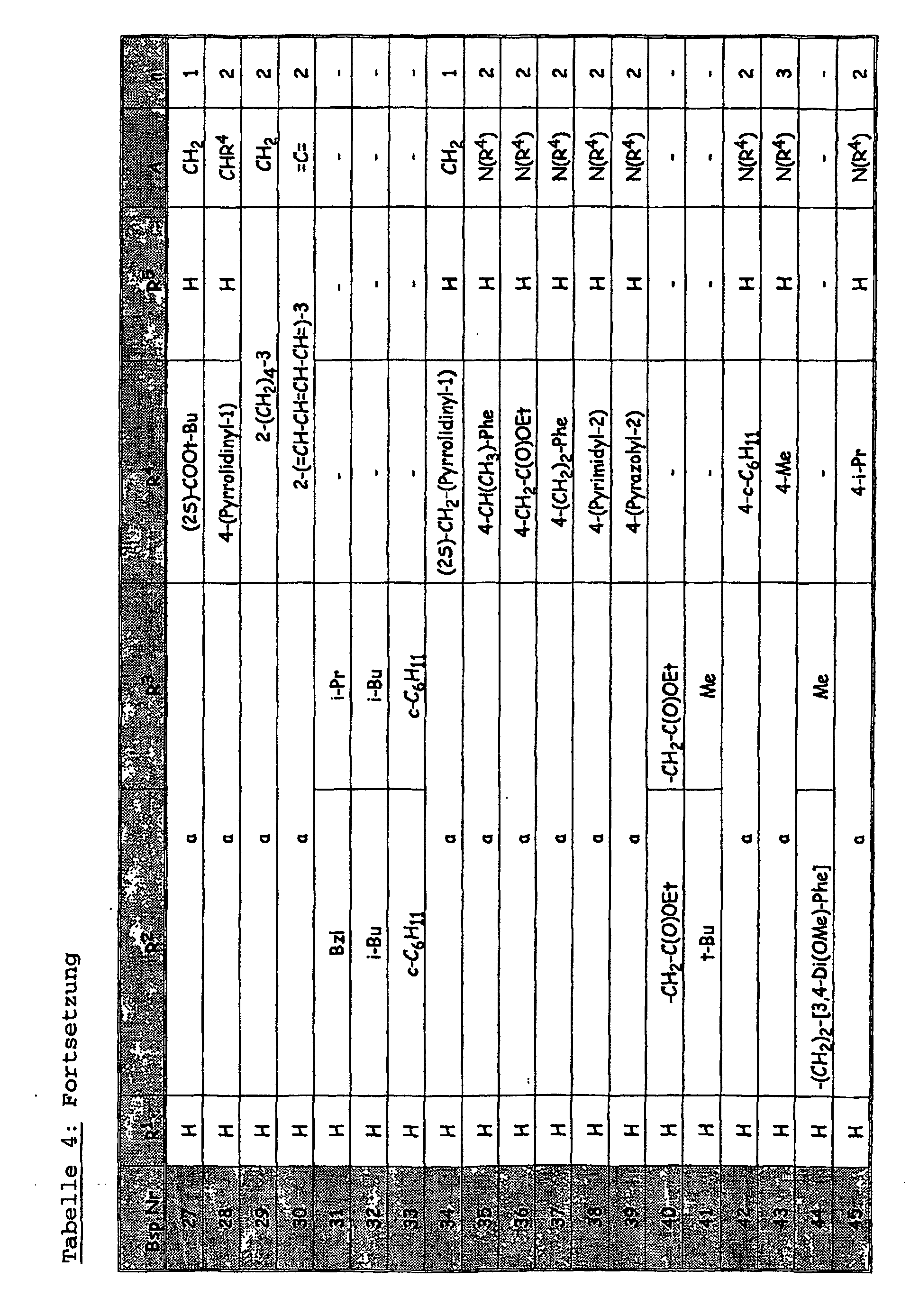

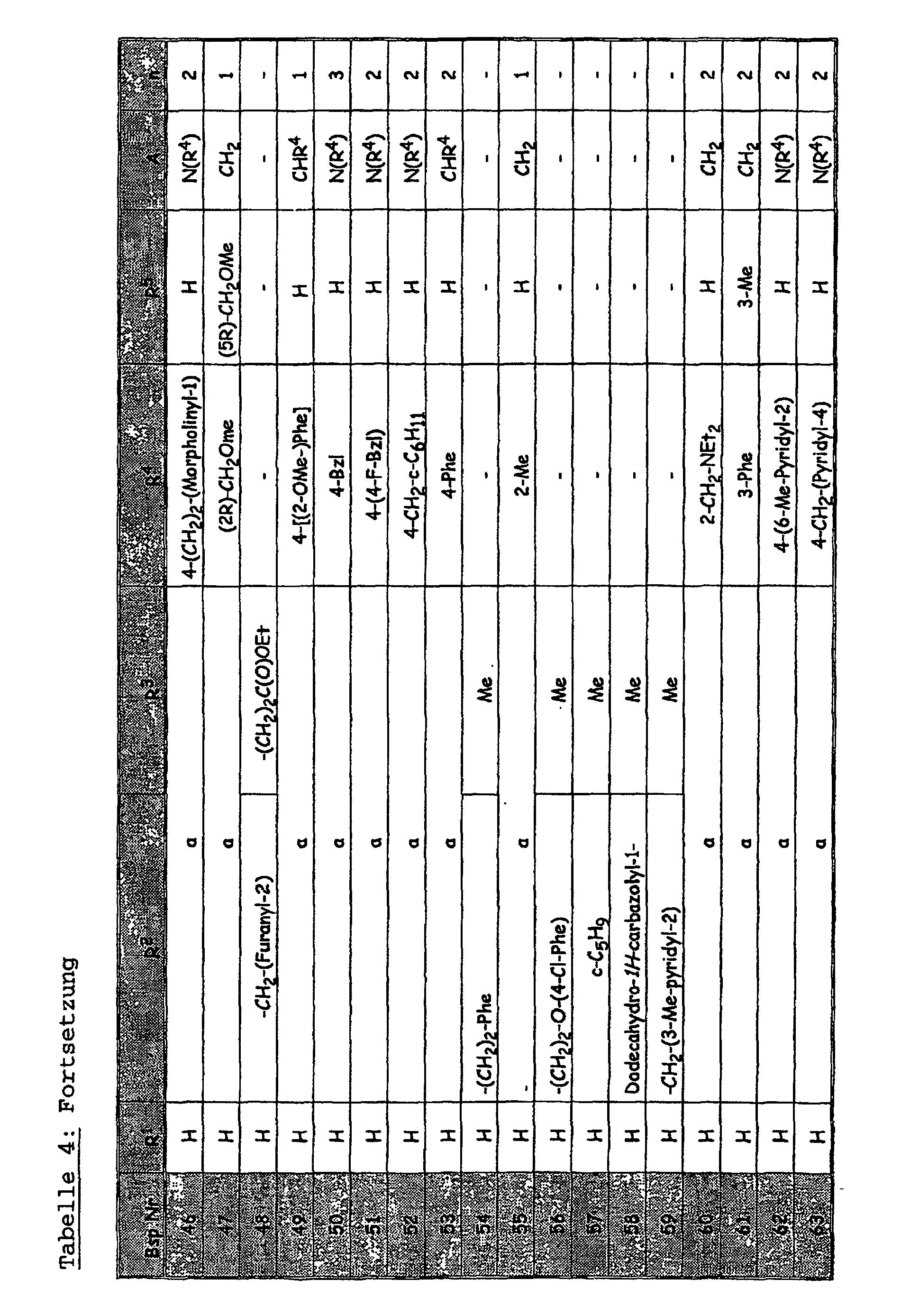

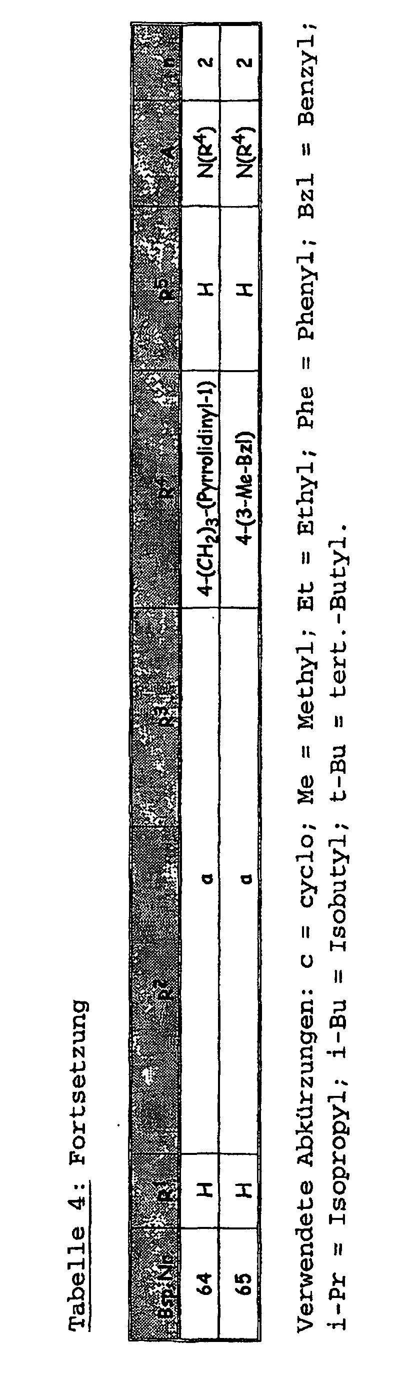

### Beispiel I:

### (2R)-1-[3,5- Bis(trifluormethyl)benzoyl]-2-(1H-indol-3-ylmethyl)-4-[(5-(morpholinomethyl)-2H-1,2,3-triazol-4-yl)methyl]piperazin enthaltende Kapseln:

Es werden Kapseln in folgender Zusammensetzung pro Kapsel hergestellt:

| | |
|---|---|
| (2R)-1-[3,5-Bis(trifluormethyl)benzoyl]-2-(1H-indol-3-ylmethyl)-4-[(5-(morpholinomethyl) -2H-1,2,3-triazol-4-yl)methyl]piperazin | 20 mg |
| Maisstärke | 60 mg |
| Milchzucker | 300 mg |
| Ethylacetat | q.s. |

Der Wirkstoff, die Maisstärke und der Milchzucker werden unter Zuhilfenahme von Ethylacetat zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkuum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verbindungen der allgemeinen Formel I, 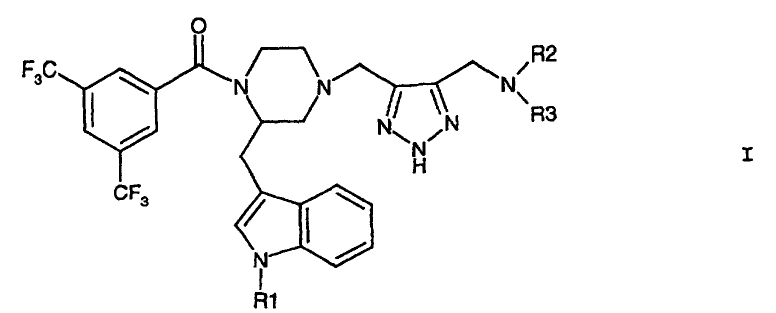 worin
R¹ Wasserstoff oder Niederalkyl bedeutet,
R² Niederalkyl, Diniederalkylaminoniederalkyl, Niederalkoxycarbonylniederalkyl, gegebenenfalls ein- oder zweifach durch Niederalkyl substituiertes Cyclo(hetero)alkyl mit 5-6 Ringatomen, welches gegebenenfalls 1-2 Doppelbindungen enthält, gegebenenfalls im (Hetero)phenylring ein- oder zweifach durch Halogen, Niederalkyl und/oder Niederalkoxy substituiertes (Hetero)phenylniederalkyl, dessen Niederalkylkette gegebenenfalls ein- oder zweifach durch Niederalkyl oder durch Spiro-C₄-C₅-alkylen substituiert ist, oder im Phenylring gegebenenfalls ein- oder zweifach durch Halogen, Niederalkyl und/oder Niederalkoxy substituiertes Phenylniederalkoxy bedeutet und
R³ Niederalkyl, Niederalkoxycarbonylniederalkyl oder gegebenenfalls ein- oder zweifach durch Niederalkyl substituiertes Cyclo(hetero)alkyl mit 5-6 Ringatomen bedeutet, oder
R² und R³ gemeinsam mit dem Stickstoff, an welchen sie gebunden sind, eine cyclische Gruppe der Formel a bilden, 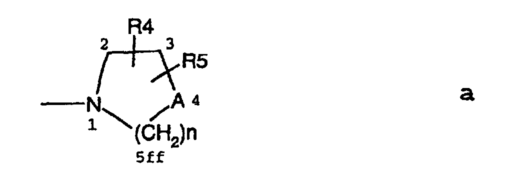 worin
A Stickstoff, Sauerstoff, Methylen oder Methyliden, dessen Doppelbindung gemeinsam mit dem benachbarten Kohlenstoff in 3-Position der Gruppe a gebildet wird, bedeutet,
n eine ganze Zahl von 1 bis 3 bedeutet,
R⁴ Wasserstoff, Niederalkyl, Niederalkoxyniederalkyl, Niederalkoxycarbonyl, Niederalkoxycarbonylniederalkyl, Diniederalkylaminoniederalkyl, gegebenenfalls ein- oder zweifach durch Halogen, Niederalkyl und/oder Niederalkoxy substituiertes (Hetero)-phenyl, gegebenenfalls im (Hetero)phenylring ein- oder zweifach durch Halogen, Niederalkyl und/oder Niederalkoxy substituiertes (Hetero)phenylniederalkyl, dessen Niederalkylkette gegebenenfalls ein - oder zweifach durch Niederalkyl substituiert ist, Cyclo(hetero)alkyl mit 5-6 Ringatomen oder Cyclo(hetero)alkylniederalkyl, dessen Cyclo(hetero)-alkylgruppe 5-6 Ringatome aufweist, bedeutet und
R⁵ Wasserstoff, Niederalkyl oder Niederalkoxyniederalkyl bedeutet, oder
R⁴ und R⁵ gemeinsam an ein Kohlenstoff der Gruppe a gebundenes Spiroethylendioxy, an zwei benachbarte Atome der Gruppe a gebundenes C₃-C₄-Alkylen oder über zwei benachbarte Kohlenstoffe der Gruppe a anelliertes Phenyl bedeuten, oder
R² und R³ gemeinsam mit dem Stickstoff, an welchen sie gebunden sind, einen Pyrrolidinring bilden, welcher zweifach durch jeweils über zwei benachbarte Kohlenstoffatome gebundenes C₄-Alkylen substituiert ist, wobei niedere Alkyl- oder Alkoxygruppen 1 bis 4 Kohlenstoffatome enthalten,
sowie physiologisch verträgliche Säureadditionssalze von Verbindungen der Formel I.

2. Verbindungen der allgemeinen Formel I gemäß Anspruch 1, worin
R¹ Wasserstoff bedeutet,
R² Niederalkyl, Diniederalkylaminoniederalkyl, Niederalkoxycarbonylniederalkyl, gegebenenfalls einfach durch Niederalkyl substituiertes Cyclo(hetero)alkykl mit 5-6 Ringatomen, gegebenenfalls im Heterophenylring ein - oder zweifach durch Niederalkyl oder Niederalkoxy substituiertes Heterophenylniederalkyl oder im Phenylring ein- oder zweifach durch Niederalkyl oder Niederalkoxy substituiertes Phenyl-C₂-C₄-alkyl bedeutet und
R³ Niederalkyl oder Niederalkoxycarbonylniederalkyl bedeutet, mit der Maßgabe, daß R² und R³ nicht gleichzeitig für Isobutyl stehen, oder
R² und R³ gemeinsam mit dem Stickstoff, an welchen sie gebunden sind, eine cyclische Gruppe der Formel a bilden,
worin
n eine ganze Zahl von 1 bis 3 bedeutet, mit der Maßgabe, daß n für 2 oder 3 steht, sofern R⁴ und R⁵ beide Wasserstoff bedeuten und gleichzeitg A für Methylen steht,
R⁴ Wasserstoff, Niederalkyl, Niederalkoxyniederalkyl, Niederalkoxycarbonylniederalkyl, Diniederalkylaminoniederalkyl, gegebenenfalls einfach durch Niederalkyl oder Niederalkoxy substituiertes (Hetero)phenyl, gegebenenfalls im (Hetero)phenylring einfach durch Halogen, Niederalkyl oder Niederalkoxy substituiertes (Hetero)phenylniederalkyl, Cyclo(hetero)alkyl mit 5-6 Ringatomen oder Cyclo(hetero)alkylniederalkyl, dessen Cyclo(hetero)alkylrest 5-6 Ringatome aufweist, bedeutet und
R⁵ Wasserstoff, Niederalkyl oder Niederalkoxyniederalkyl bedeutet, mit der Maßgabe, daß R⁴ und R⁵ nicht an dasselbe Ringatom der Gruppe a gebunden sind, oder
R⁴ und R⁵ gemeinsam an einen Kohlenstoff der Gruppe a gebundenes Spiroethylendioxy oder an zwei benachbarte Ringatome der Gruppe a gebundenes C₃-C₄Alkylen bedeuten.

3. Verbindungen der allgemeinen Formel I nach Anspruch 1 oder 2, worin der den 1H-Indol-3-yl-methylrest tragende Kohlenstoff C-2 des Piperazinringes die R-Konfiguration aufweist.

4. Verbindungen der allgemeinen Formel I gemäß einem der vorstehenden Ansprüche, worin eine gegebenenfalls vorhandene Gruppe a für durch R⁴ und R⁵ substituiertes Pyrrolidin steht, wobei R⁴ und R⁵ nicht beide gleichzeitig Wasserstoff bedeuten, oder für jeweils durch R⁴ und R⁵ substituiertes 2,5-Dihydropyrrol, Piperidin, Piperazin, Morpholin oder Diazepan steht.

5. (2R)-1-[3,5-Bis(trifluormethyl)benzoyl]-2-(1H-indol-3-ylmethyl)-4-{[5-(morpholinomethyl)-2H-1,2,3-triazol-4-yl] methyl}piperazin gemäß Anspruch 4.

6. (2R)-1-[3,5-Bis(trifluormethyl)benzoyl]-2-(1H-indol-3-ylmethyl)-4-{[5-(morpholinomethyl)-2H-1,2,3-triazol-4-yl]methyl}piperazin-dihydrochlotid gemäß Anspruch 5.

7. Arzneimittel, enthaltend eine pharmakologisch wirksame Menge einer Verbindung gemäß Anspruch 1 und übliche pharmazeutische Hilfs- und/oder Trägerstoffe.

8. Verfahren zur Herstellung von Verbindungen der allgemeinen Formel I, 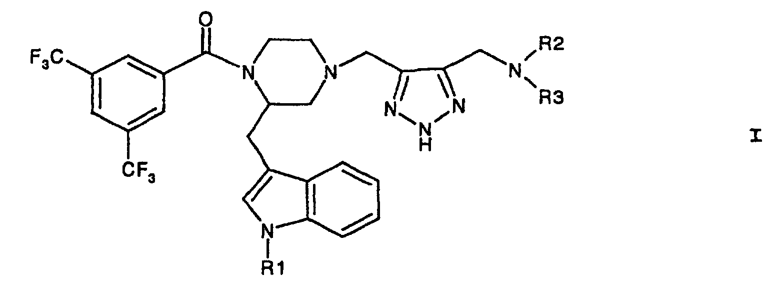 worin
R¹ Wasserstoff oder Niederalkyl bedeutet,
R² Niederalkyl, Diniederalkylaminoniederalkyl, Niederalkoxycarbonylniederalkyl, gegebenenfalls ein- oder zweifach durch Niederalkyl substituiertes Cyclo(hetero)-alkyl mit 5-6 Ringatomen, welches gegebenenfalls 1-2 Doppelbindungen enthält, gegebenenfalls im (Hetero)-phenylring ein- oder zweifach durch Halogen, Niederalkyl und/oder Niederalkoxy substituiertes (Hetero)-phenylniederalkyl, dessen Niederalkylkette gegebenenfalls ein- oder zweifach durch Niederalkyl oder durch Spiro-C₄-C₅-alkylen substituiert ist, oder im Phenylring gegebenenfalls ein- oder zweifach durch Halogen, Niederalkyl und/oder Niederalkoxy substituiertes Phenylniederalkoxy bedeutet und
R³ Niederalkyl, Niederalkoxycarbonylniederalkyl oder gegebenenfalls ein- oder zweifach durch Niederalkyl substituiertes Cyclo(hetero)alkyl mit 5-6 Ringatomen bedeutet, oder
R² und R³ gemeinsam mit dem Stickstoff, an welchen sie gebunden sind, eine cyclische Gruppe der Formel a bilden, 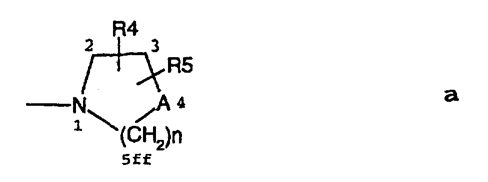 worin
A Stickstoff, Sauerstoff, Methylen oder Methyliden, dessen Doppelbindung gemeinsam mit dem benachbarten Kohlenstoff in 3-Position der Gruppe a gebildet wird, bedeutet,
n eine ganze Zahl von 1 bis 3 bedeutet,
R⁴ Wasserstoff, Niederalkyl, Niederalkoxyniederalkyl, Niederalkoxycarbonyl, Niederalkoxycarbonylniederalkyl, Diniederalkylaminoniederalkyl, gegebenenfalls ein- oder zweifach durch Halogen, Niederalkyl und/oder Niederalkoxy substituiertes (Hetero)phenyl, gegebenenfalls im (Hetero)phenylring ein - oder zweifach durch Halogen, Niederalkyl und/oder Niederalkoxy substituiertes (Hetero)phenylniederalkyl, dessen Niederalkylkette gegebenenfalls ein- oder zweifach durch Niederalkyl substituiert ist, Cyclo(hetero)alkyl mit 5-6 Ringatomen oder Cyclo(hetero)alkylniederalkyl, dessen Cyclo(hetero)alkylgruppe 5-6 Ringatome aufweist, bedeutet und
R⁵ Wasserstoff, Niederalkyl oder Niederalkoxyniederalkyl bedeutet, oder
R⁴ und R⁵ gemeinsam an ein Kohlenstoff der Gruppe a gebundenes Spiroethylendioxy, an zwei benachbarte Atome der Gruppe a gebundenes C₃-C₄-Alkylen oder über zwei benachbarte Kohlenstoffe der Gruppe a anelliertes Phenyl bedeuten, oder
R² und R³ gemeinsam mit dem Stickstoff, an welchen sie gebunden sind, einen Pyrrolidinring bilden, welcher zweifach durch jeweils über zwei benachbarte Kohlenstoffatome gebundenes C₄-Alkylen substituiert ist, wobei niedere Alkyl- oder Alkoxygruppen 1 bis 4 Kohlenstoffatome enthalten,
sowie deren physiologisch verträglichen Saüreadditionssalzen, **dadurch gekennzeichnet, daß** man
a) eine Verbindung der allgemeinen Formel II, 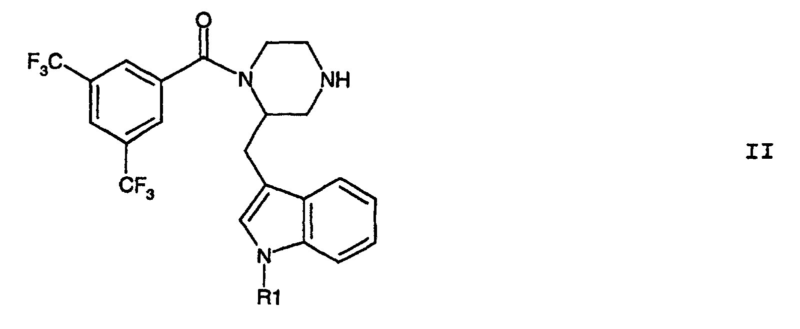 worin R¹ obige Bedeutung besitzt, mit einer Verbindung der allgemeinen Formel III, 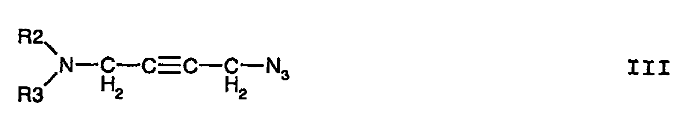 worin R² und R³ obige Bedeutungen besitzen, und worin allenfalls vorhandene reaktive Gruppen durch geeignete Schutzgruppen blockiert sind, umsetzt, oder
b) eine Verbindung der allgemeinen Formel IV 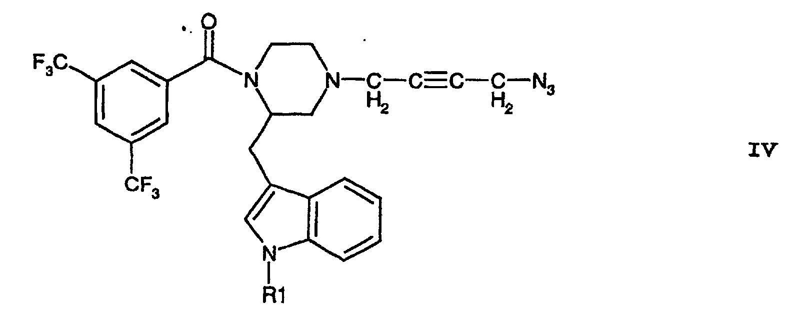 worin R¹ obige Bedeutung besitzt, mit einer Verbindung der allgemeinen Formel V, 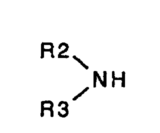 worin R² und R³ obige Bedeutungen besitzen, und worin allenfalls vorhandene reaktive Gruppen durch geeignete Schutzgruppen blockiert sind, umsetzt,
und allenfalls vorhandene Schutzgruppen nachfolgend wieder abspaltet und eine erhaltene Verbindung der Formel I gewünschtenfalls in ihr Säureadditionssalz überführ oder ein Säureadditionssalz in eine freie Verbindung der Formel I überführt.

9. Verbindungen der allgemeinen Formel IV 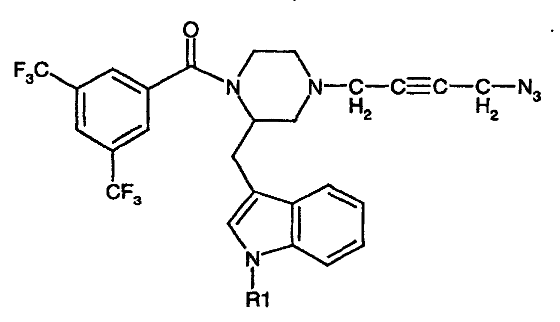 worin
R¹ Wasserstoff oder Niederalkyl mit 1 bis 4 Kohlenstoffatome bedeutet.

## Claims

1. Compounds of the general formula I, 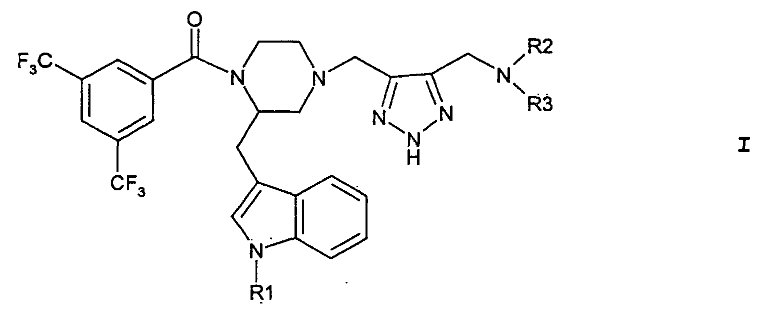 wherein
R¹ is hydrogen or lower alkyl,
R² is lower alkyl, di-lower-alkylamino lower alkyl, loweralkoxycarbonyl lower alkyl; cyclo(hetero)alkyl having 5-6 ring atoms, which may optionally be substituted once or twice by lower alkyl and which optionally contains 1-2 double bonds; (hetero)phenyl lower alkyl optionally substituted once or twice in the (hetero)phenyl ring by halogen, lower alkyl and/or lower alkoxy, the lower-alkyl chain of which (hetero)phenyl lower alkyl is optionally substituted once or twice by lower alkyl or by spiro-C₄-C₅alkylene; or phenyl lower alkoxy optionally substituted once or twice in the phenyl ring by halogen, lower alkyl and/or lower alkoxy, and
R³ is lower alkyl, lower-alkoxycarbonyl lower alkyl or cyclo(hetero)alkyl with 5-6 ring atoms which is optionally substituted once or twice by lower alkyl, or
R² and R³, together with the nitrogen to which they are bonded, form a cyclic group of formula a, 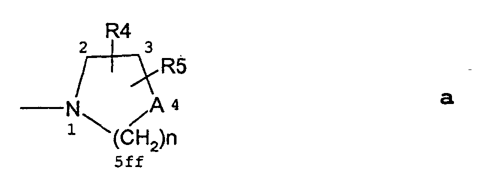 wherein
A is nitrogen, oxygen, methylene or methylidene, the double bond of which, together with the adjacent carbon, is formed in position 3 of group a,
n is a whole number from 1 to 3,
R⁴ is hydrogen, lower alkyl, lower-alkoxy lower alkyl, lower alkoxycarbonyl, lower-alkoxycarbonyl lower alkyl, di-lower-alkylamino lower alkyl; (hetero)phenyl optionally substituted once or twice by halogen, lower alkyl and/or lower alkoxy; (hetero)phenyl lower alkyl optionally substituted once or twice in the (hetero)phenyl ring by halogen, lower alkyl and/or lower alkoxy, the lower-alkyl chain of which (hetero)phenyl lower alkyl is optionally substituted once or twice by lower alkyl; cyclo(hetero)alkyl with 5-6 ring atoms; or cyclo(hetero)alkyl lower alkyl, the cyclo(hetero)alkyl group of which has 5-6 ring atoms, and
R⁵ is hydrogen, lower alkyl or lower-alkoxy lower alkyl, or
R⁴ and R⁵ together are spiroethylenedioxy bonded to a carbon of group a; C₃-C₄-alkylene bonded to two adjacent atoms of group a; or phenyl fused via two adjacent carbons of group a, or
R² and R³, together with the nitrogen to which they are bonded, form a pyrrolidine ring which is substituted twice by C₄-alkylene which is bonded each time via two adjacent carbon atoms,
lower alkyl or alkoxy groups containing 1 to 4 carbon atoms, and physiologically compatible acid addition salts of compounds of Formula I.

2. Compounds of the general formula I according to Claim 1, wherein
R¹ is hydrogen,
R² is lower alkyl, di-lower-alkylamino lower alkyl, lower-alkoxycarbonyl lower alkyl; cyclo(hetero)alkyl with 5-6 ring atoms, optionally substituted once by lower alkyl; heterophenyl lower alkyl optionally substituted once or twice in the heterophenyl ring by lower alkyl or lower alkoxy, or phenyl-C₂-C₄-alkyl substituted once or twice in the phenyl ring by lower alkyl or lower alkoxy, and
R³ is lower alkyl or lower-alkoxycarbonyl lower alkyl, with the proviso that R² and R³ do not simultaneously stand for isobutyl, or
R² and R³, together with the nitrogen to which they are bonded, form a cyclic group of formula a, wherein
n is a whole number from 1 to 3, with the proviso that n stands for 2 or 3, provided that R⁴ and R⁵ are both hydrogen and at the same time A stands for methylene,
R⁴ is hydrogen, lower alkyl, lower-alkoxy lower alkyl, lower-alkoxycarbonyl lower alkyl, di-lower-alkylamino lower alkyl; (hetero)phenyl optionally substituted once by lower alkyl or lower alkoxy; (hetero)phenyl lower alkyl optionally substituted once in the (hetero)phenyl ring by halogen, lower alkyl or lower alkoxy; cyclo(hetero)alkyl with 5-6 ring atoms; or cyclo(hetero)alkyl lower alkyl, the cyclo(hetero)alkyl radical of which has 5-6 ring atoms, and
R⁵ is hydrogen, lower alkyl or lower alkoxy lower alkyl, with the proviso that R⁴ and R⁵ are not bonded to the same ring atom of group a, or
R⁴ and R⁵ together are spiroethylenedioxy bonded to a carbon of group a; or C₃-C₄-alkylene bonded to two adjacent ring atoms of group a.

3. Compounds of the general formula I according to Claim 1 or 2, wherein the carbon C-2 of the piperazine ring which bears the 1H-indol-3-yl-methy radical is in the R configuration.

4. Compounds of the general formula I according to one of the preceding claims, wherein an optionally present group a stands for pyrrolidine substituted by R⁴ and R⁵, wherein R⁴ and R⁵ are not both simultaneously hydrogen, or for 2,5-dihydropyrrole, piperidine, piperazine, morpholine or diazepan, each substituted by R⁴ and R⁵.

5. (2R)-1-[3,5-Bis(trifluoromethyl)benzoyl]-2-(1H-indol-3-ylmethyl)-4-{[5-(morpholinomethyl)-2H-1,2,3-triazol-4-yl]methyl}piperazine according to Claim 4.

6. (2R)-1-[3,5-Bis(trifluoromethyl)benzoyl]-2-(1H-indol-3-ylmethyl)-4-{[5-(morpholinomethyl)-2H-1,2,3-triazol-4-yl]methyl}piperazine dihydrochloride according to Claim 5.

7. Medicament, containing a pharmacologically active quantity of a compound according to Claim 1 and conventional pharmaceutical auxiliaries and/or carriers.

8. A process for the preparation of compounds of the general formula I, 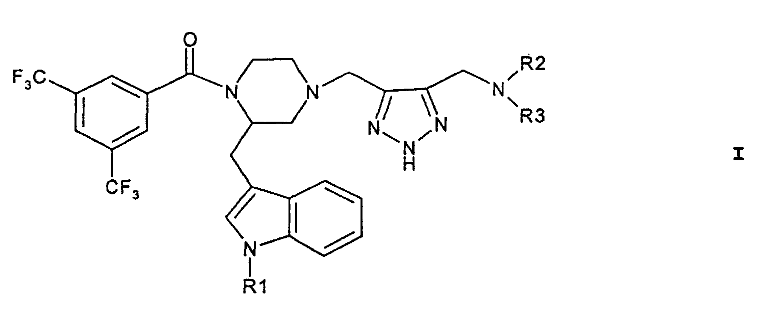 wherein
R¹ is hydrogen or lower alkyl,
R² is lower alkyl, di-lower-alkylamino lower alkyl, lower-alkoxycarbonyl lower alkyl; cyclo(hetero)alkyl having 5-6 ring atoms, which may optionally be substituted once or twice by lower alkyl and which optionally contains 1-2 double bonds; (hetero)phenyl lower alkyl optionally substituted once or twice in the (hetero)phenyl ring by halogen, lower alkyl and/or lower alkoxy, the lower-alkyl chain of which (hetero)phenyl lower alkyl is optionally substituted once or twice by lower alkyl or by spiro-C₄-C₅-alkylene; or phenyl lower alkoxy optionally substituted once or twice in the phenyl ring by halogen, lower alkyl and/or lower alkoxy, and
R³ is lower alkyl, lower-alkoxycarbonyl lower alkyl or cyclo(hetero)alkyl with 5-6 ring atoms which is optionally substituted once or twice by lower alkyl, or
R² and R³, together with the nitrogen to which they are bonded, form a cyclic group of formula a, 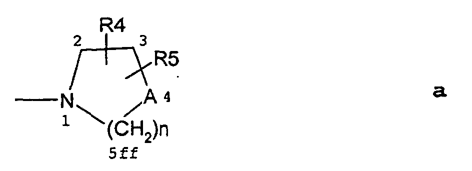 wherein
A is nitrogen, oxygen, methylene or methylidene, the double bond of which, together with the adjacent carbon, is formed in position 3 of group a,
n is a whole number from 1 to 3,
R⁴ is hydrogen, lower alkyl, lower-alkoxy lower alkyl, lower alkoxycarbonyl, lower-alkoxycarbonyl lower alkyl, di-lower-alkylamino lower alkyl, (hetero)phenyl optionally substituted once or twice by halogen, lower alkyl and/or lower alkoxy; (hetero)phenyl lower alkyl optionally substituted once or twice in the (hetero)phenyl ring by halogen, lower alkyl and/or lower alkoxy, the lower-alkyl chain of which (hetero)phenyl lower alkyl is optionally substituted once or twice by lower alkyl; cyclo(hetero)alkyl with 5-6 ring atoms; or cyclo(hetero)alkyl lower alkyl, the cyclo(hetero)alkyl group of which has 5-6 ring atoms, and
R⁵ is hydrogen, lower alkyl or lower-alkoxy lower alkyl, or
R⁴ and R⁵ together are spiroethylenedioxy bonded to a carbon of group a; C₃-C₄-alkylene bonded to two adjacent atoms of group a; or phenyl fused via two adjacent carbons of group a, or
R² and R³, together with the nitrogen to which they are bonded, form a pyrrolidine ring which is substituted twice by C₄-alkylene which is bonded each time via two adjacent carbon atoms,
lower alkyl or alkoxy groups containing 1 to 4 carbon atoms, and their physiologically compatible acid addition salts, **characterised in that**
a) a compound of the general formula II, 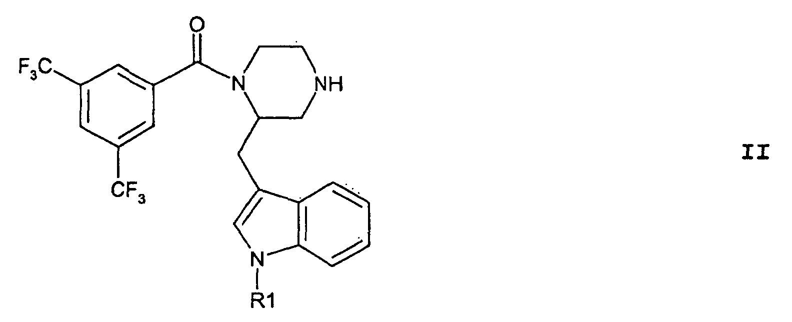 wherein R¹ has the above meaning, is reacted with a compound of the general formula III, 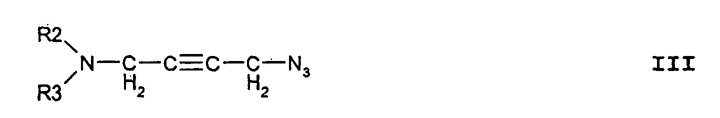 wherein R² and R³ have the above meanings, and wherein any reactive groups present are blocked by suitable protective groups, or
b) a compound of the general formula IV 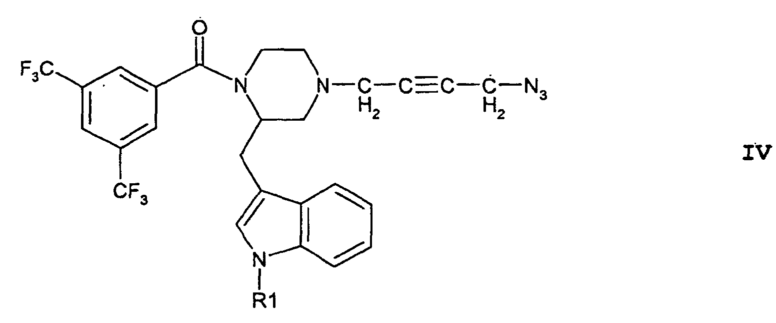 wherein R¹ has the above meaning, is reacted with a compound of the general formula V, 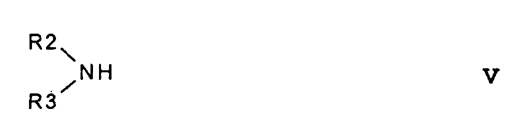 wherein R² and R³ have the above meanings, and wherein any reactive groups present are blocked by suitable protective groups,
and any protective groups present are subsequently cleaved off again and a resulting compound of Formula I if desired is converted into its acid addition salt or an acid addition salt is converted into a free compound of Formula I.

9. Compounds of the general formula IV 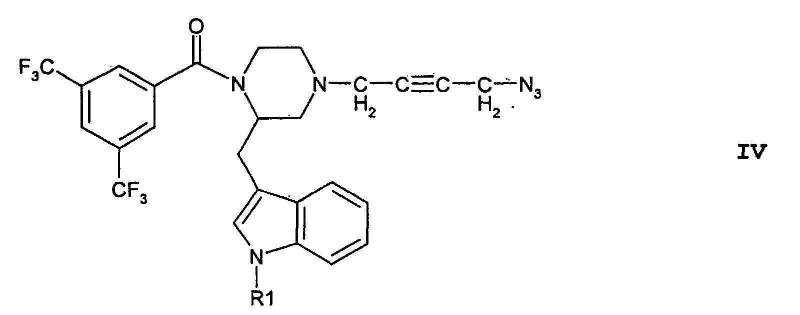 wherein
R¹ is hydrogen or lower alkyl with 1 to 4 carbon atoms.

## Revendications

1. Composés de formule générale I, 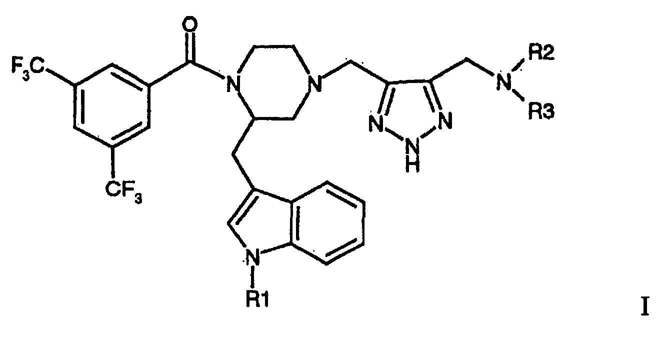 dans laquelle
R¹ représente l'hydrogène ou un alkyle inférieur,
R² représente un alkyle inférieur, un di(alkyle inférieur)-amino-(alkyle inférieur), un (alcoxy inférieur)-carbonyl-alkyle inférieur, un cyclo(hétéro)alkyle ayant 5 à 6 atomes dans le cycle, éventuellement mono- ou bisubstitué par un allyle inférieur, qui contient le cas échéant 1 à 2 doubles liaisons, un (hétéro)phényl-(alkyle inférieur) éventuellement mono- ou bisubstitué dans le noyau (hétéro)phényle par un halogène, un alkyle inférieur et/ou un alcoxy inférieur, et dont la chaîne alkyle inférieur est éventuellement mono- ou bisubstituée par un alkyle inférieur ou par un spiro-alkylène en C₄ à C₅, ou un phénylalcoxy inférieur, éventuellement mono- ou bisubstitué dans le noyau phényle par un halogène, un alkyle inférieur et/ou un alcoxy inférieur, et
R³ représente un alkyle inférieur, un (alcoxy inférieur)-carbonyl-alkyle inférieur ou un cyclo(hétéro)alkyle éventuellement mono- ou bisubstitué par un alkyle inférieur et comportant de 5 à 6 atomes dans le cycle, ou
R² et R³ représentent ensemble avec l'azote auquel il sont liés un groupe cyclique de formule a, 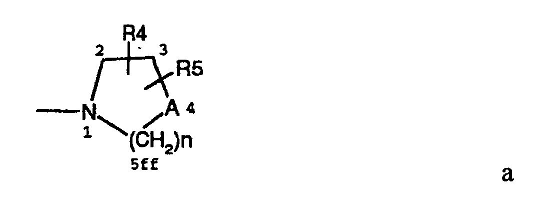 dans laquelle
A représente l'azote, l'oxygène, un méthylène ou un méthylidène, dont la double liaison est formée en commun avec l'atome de carbone voisin en position 3 du groupe a,
n représente un nombre entier de 1 à 3,
R⁴ représente l'hydrogène, un alkyle inférieur, un alcoxy inférieur-alkyle inférieur, un (alcoxy inférieur)-carbonyle, un alcoxy inférieur-carbonyl-(alkyle inférieur), un di(alkyle inférieur)-amino-alkyle inférieur, un (hétéro)phényle éventuellement mono- ou bisubstitué par un halogène, un alkyle inférieur et/ou un alcoxy inférieur, un (hétéro)phényl(alkyle inférieur) éventuellement mono- ou bisubstitué dans le noyau (hétéro)phényle par un halogène, un alkyle inférieur et/ou un alcoxy inférieur, et dont la chaîne alkyle inférieur est éventuellement mono- ou bisubstituée par un alkyle inférieur, un cyclo(hétéro)alkyle comportant 5 à 6 atomes dans le cycle ou un cyclo(hétéro)alkyl(alkyle inférieur) dont le groupe cyclo(hétéro)alkyle présente 5 à 6 atomes dans le cycle, et
R⁵ représente l'hydrogène, un alkyle inférieur ou un alcoxy inférieur-alkyle inférieur, ou
R⁴ et R⁵ ensemble représentent un spiroéthylène-dioxy lié à un atome de carbone du groupe a, un alkylène en C₃ à C₄ lié à deux atomes voisins du groupe a ou un phényle condensé par deux atomes de carbone voisins du groupe a, ou
R² et R³ représentent ensemble avec l'atome d'azote auquel ils sont liés un noyau pyrrolidine qui est bisubstitué par respectivement un alkylène en C₄ lié par l'intermédiaire de deux atomes de carbone voisins,
les groupes alkyle ou alcoxy inférieurs contenant 1 à 4 atomes de carbone, ainsi que les sels d'addition acides physiologiquement acceptables des composés de formule I.

2. Composés de formule générale I selon la revendication 1, dans laquelle
R¹ représente l'hydrogène,
R² représente un alkyle inférieur, un di(alkyle inférieur)-amino-alkyle inférieur, un (alcoxy inférieur)-carbonyl-alkyle inférieur, un cyclo(hétéro)alkyle ayant 5 à 6 atomes dans le cycle, éventuellement monosubstitué par un alkyle inférieur, un hétérophényl alkyle inférieur éventuellement mono- ou bisubstitué dans le noyau hétérophényle par un alkyle inférieur ou un alcoxy inférieur, ou un phényl-alkyle en C₂ à C₄ mono- ou bisubstitué dans le noyau phényle par un alkyle inférieur ou un alcoxy inférieur, et
R³ représente un alkyle inférieur ou un (alcoxy inférieur)-carbonyl-alkyle inférieur, sous réserve que R² et R³ ne représentent pas simultanément un isobutyle, ou
R² et R³ représentent ensemble avec l'azote auquel ils sont liés un groupe cyclique de formule a, dans laquelle
n représente un nombre entier de 1 à 3, sous réserve que n vaut 2 ou 3 lorsque R⁴ et R⁵ représentent tous deux l'hydrogène et A représente simultanément un méthylène,
R⁴ représente l'hydrogène, un alkyle inférieur, un alcoxy inférieur-alkyle inférieur, un alcoxy inférieur-carbonyl-(alkyle inférieur), un di(alkyle inférieur)-amino-alkyle inférieur, un (hétéro)phényle éventuellement monosubstitué par un alkyle inférieur ou un alcoxy inférieur, un (hétéro)phényl(alkyle inférieur) éventuellement monosubstitué dans le noyau (hétéro)phényle par un halogène, un alkyle inférieur ou un alcoxy inférieur, un cyclo(hétéro)alkyle comportant 5 à 6 atomes dans le cycle ou un cyclo(hétéro)alkyl(alkyle inférieur) dont le groupe cyclo(hétéro)alkyle comporte 5 à 6 atomes dans le cycle, et
R⁵ représente l'hydrogène, un alkyle inférieur ou un alcoxy inférieur-alkyle inférieur, sous réserve que R⁴ et R⁵ ne soient pas liés au même atome du cycle du groupe a, ou
R⁴ et R⁵ ensemble représentent un spiroéthylène-dioxy lié à un atome de carbone du groupe a ou un alkylène en C₃ à C₄ lié à deux atomes cycliques voisins du groupe a.

3. Composés de formule générale I selon la revendication 1 ou 2, dans laquelle l'atome de carbone C-2 du noyau pipérazine portant le radical 1H-indol-3-yl-méthyle présente la configuration R.

4. Composés de formule générale I selon l'une des revendications précédentes, dans laquelle un groupe a éventuellement présent représente une pyrrolidine substituée par R⁴ et R⁵, où R⁴ et R⁵ ne représentent pas tous deux simultanément l'hydrogène, ou bien un 2,5-dihydropyrrole, une pipéridine, une pipérazine, une morpholine ou un diazépam respectivement substitué par R⁴ et R⁵.

5. (2R)-1-[3,5-bis(trifluorométhyl)benzoyl]-2-(1H-indol-3-yl-méthyl)-4-{[(5-(morpholinométhyl)-2H-1,2,3-triazol-4-yl]méthyl}pipérazine selon la revendication 4.

6. Dichlorhydrate de (2R)-1-[3,5-bis(trifluorométhyl)benzoyl]-2-(1H-indol-3-ylméthyl)-4-{[5-(morpholinométhyl)-2H-1,2,3-triazol-4-yl]-méthyl}pipérazine selon la revendication 5.

7. Médicament contenant une quantité pharmacologiquement efficace d'un composé selon la revendication 1 et des adjuvants et/ou supports pharmaceutiques habituels.

8. Procédé de préparation de composés de formule générale I, 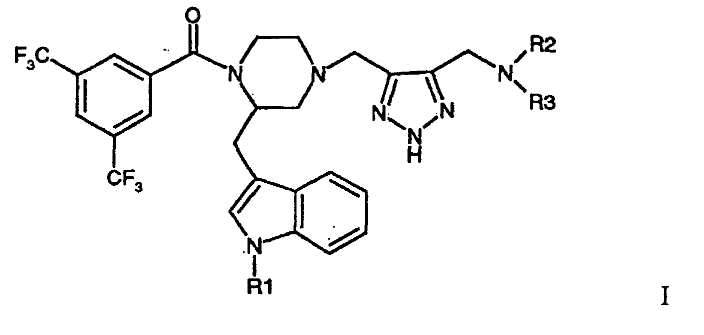 dans laquelle
R¹ représente l'hydrogène ou un alkyle inférieure,
R² représente un alkyle inférieur, un di(alkyle inférieur) amino alkyle inférieur, un alcoxy inférieur-carbonyl-alkyle inférieur, un cyclo(hétéro)alkyle ayant 5 à 6 atomes dans le cycle, éventuellement mono- ou bisubstitué par un alkyle inférieur, qui contient le cas échéant 1 à 2 doubles liaisons, un (hétéro)phényl-alkyle inférieur éventuellement mono- ou bisubstitué dans le noyau (hétéro)phényle par un halogène, un alkyle inférieur et/ou un alcoxy inférieur, et dont la chaîne alkyle inférieur est éventuellement mono- ou bisubstituée par un alkyle inférieur ou par un spiro-alkylène en C₄ à C₅, ou un phénylalcoxy inférieur éventuellement mono- ou bisubstitué dans le noyau phényle par un halogène, un alkyle inférieur et/ou un alcoxy inférieur, et
R³ représente un alkyle inférieur, un alcoxy inférieur carbonyl-alkyle inférieur ou un cyclo(hétéro)alkyle éventuellement mono- ou bisubstitué par un alkyle inférieur et comportant de 5 à 6 atomes dans le cycle, ou
R² et R³ représentent ensemble avec l'azote auquel ils sont liés un groupe cyclique de formule a, 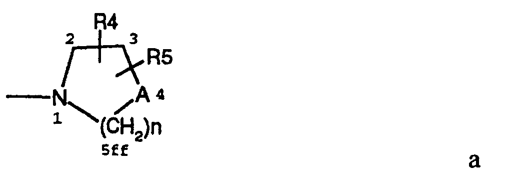 dans laquelle
A représente l'azote, l'oxygène, un méthylène ou un méthylidène, dont la double liaison est formée en commun avec l'atome de carbone voisin en position 3 du groupe a,
n représente un nombre entier de 1 à 3,
R⁴ représente l'hydrogène, un alkyle inférieur, un alcoxy inférieur-alkyle inférieur, un alcoxy inférieur carbonyle, un alcoxy inférieur-carbonyl-alkyle inférieur, un di(alkyle inférieur)-amino-alkyle inférieur, un (hétéro)phényle éventuellement mono- ou bisubstitué par un halogène, un alkyle inférieur et/ou un alcoxy inférieur, un (hétéro)phénylalkyle inférieur éventuellement mono- ou bisubstitué dans le noyau (hétéro)phényle par un halogène, un alkyle inférieur et/ou un alcoxy inférieur, et dont la chaîne alkyle inférieur est éventuellement mono- ou bisubstituée par un alkyle inférieur, un cyclo(hétéro)-alkyl-alkyle comportant 5 à 6 atomes dans le cycle ou un cyclo(hétéro)alkyle inférieur dont le groupe cyclo(hétéro)alkyle présente 5 à 6 atomes dans le cycle, et
R⁵ représente l'hydrogène, un alkyle inférieur ou un alcoxy inférieur-alkyle inférieur, ou
R⁴ et R⁵ ensemble représentent un spiroéthylène-dioxy lié sur un atome de carbone du groupe a, un alkylène en C₃ à C₄ lié à deux atomes voisins du groupe a ou un phényle condensé par deux atomes de carbone voisins du groupe a, ou
R² et R³ représentent ensemble avec l'atome d'azote auquel ils sont liés un noyau pyrrolidine qui est bisubstitué par respectivement un alkylène en C₄ lié par l'intermédiaire de deux atomes de carbone voisins,
les groupes alkyle ou alcoxy inférieurs contenant 1 à 4 atomes de carbone, ainsi que leurs sels d'addition acides physiologiquement acceptables,
**caractérisé en ce que**
a) on fait réagir un composé de formule générale II, 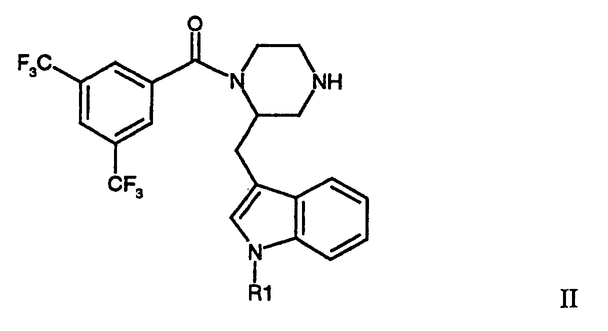 dans laquelle R¹ possède la signification ci-dessus, avec un composé de formule générale III, 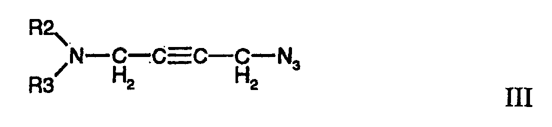 dans laquelle R² et R³ possèdent les significations ci-dessus, et dans laquelle les groupes réactifs éventuellement présents sont bloqués par des groupes protecteurs appropriés, ou
b) on fait réagir un composé de formule générale IV 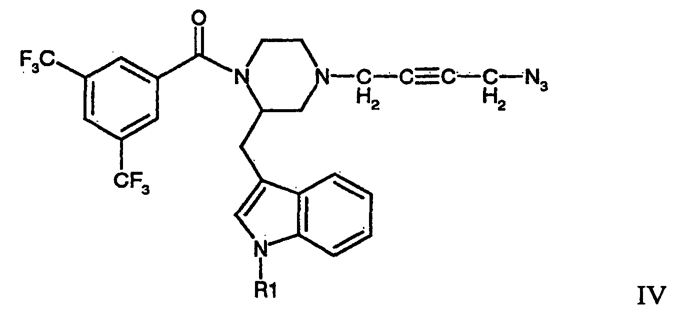 dans laquelle R¹ possède la signification ci-dessus, avec un composé de formule générale V 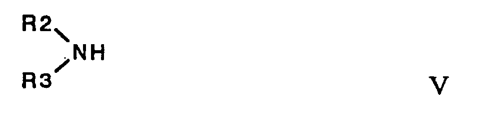 dans laquelle R² et R³ possèdent les significations données ci-dessus et dans laquelle les groupes réactifs éventuellement présents sont bloqués par des groupes protecteurs appropriés,
et on sépare ensuite à nouveau les groupes protecteurs éventuellement présents et on transforme un composé de formule I obtenu, si on le désire, en son sel d'addition d'acide, ou on transforme un sel d'addition d'acide en un composé libre de formule I.

9. Composés de formule générale IV 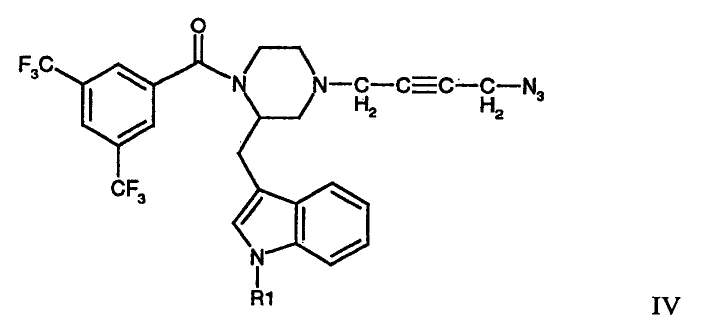 dans laquelle
R¹ est l'hydrogène ou un alkyle inférieur avec 1 à 4 atomes de carbones.
